# FASCICULE DE BREVET EUROPEEN

(11) **EP 4 340 839 B1**
(45) Date de publication et mention de la délivrance du brevet: **12.02.2025**
(21) Numéro de dépôt: 22732295.5
(22) Date de dépôt: 23.05.2022
(51) Int. Cl.: A61K 31/496, A61K 45/06, A61P 31/12, A61P 31/14

(54) **NOUVEAUX DERIVES AZAINDOLE EN TANT QU'AGENTS ANTIVIRAUX**
NEUE AZAINDOLDERIVATE ALS ANTIVIRALE MITTEL
NOVEL AZAINDOLE DERIVATIVES AS ANTIVIRAL AGENTS

(30) Priorité: 21.05.2021 FR 2105359
(43) Date de publication de la demande: 27.03.2024
(73) Titulaire: Centre National de la Recherche Scientifique (CNRS), 75016 Paris (FR); Universite de Montpellier (UM), 34090 Montpellier (FR)
(72) Inventeur: BRIANT, Laurence, 30660 GALLARGUES-LE-MONTUEUX (FR); BERNARD, Eric, 34090 MONTPELLIER (FR); CLOP, Camille, 30210 FOURNÈS (FR)
(74) Mandataire: Novagraaf Technologies
(86) Numéro de dépôt international: PCT/FR2022/050979
(87) Numéro de publication internationale: WO 2022/243650

(56) Documents cités:
- KR-A- 20120 061 011
- US-A1- 2003 207 910
- JI WEN-TSAI ET AL: "PI3K-Akt Signaling and Viral Infection", RECENT PATENTS ON BIOTECHNOLOGY, vol. 2, no. 3, 1 November 2008 (2008-11-01), NL, pages 218 - 226, XP055883954, ISSN: 1872-2083, DOI: 10.2174/187220808786241042

## Description

### DOMAINE DE L'INVENTION

La présente invention concerne des composés dérivés de 7-azaindole utiles en tant qu'inhibiteurs des kinases AXL pour le traitement des infections Est également décrit leur procédé de préparation.

### ARRIERE-PLAN TECHNOLOGIQUE

AXL est un Récepteur Tyrosine Kinase (RTK) appartenant à la famille TAM composée de TYRO-3, AXL et MER. Initialement découvert et mis en évidence chez des patients atteints de leucémie myéloïde chronique (LMC), l'implication du récepteur AXL dans des infections virales comme par exemple la dengue, le virus Zika, le Chikungunya ou encore la fièvre Ebola a été décrite dans la littérature (Chen, J. et al. Nature Microbiology, 2018, 3, pp.302-309 ; Fedeli, C. et al. Journal of Virology, 2018, 92:e01613-17 ; Hastings, A.K. et al., iScience, 2019, 13 pp.339-350 ; Meertens, L. et al., Cell Reports, 2017, 18, 3, pp.324-333) . Ce récepteur, en interagissant via l'adaptateur Gas6 avec les phosphatidylsérines présentes dans les enveloppes virales, favorise l'attachement des virus enveloppés à leur cible cellulaire. AXL stimule ainsi l'endocytose virale. L'engagement d'AXL au cours de cette interaction, stimule la phosphorylation du domaine intracytoplasmique d'AXL et active les voies de signalisation associées. Ces signaux neutralisent la production d'interférons et les réponses antivirales associées, favorisant l'échappement de ces virus au contrôle immunitaire de l'hôte.

A ce jour, il existe plusieurs inhibiteurs de kinase actifs sur AXL (Myers et al., J. Med. Chem. (2015), 59(8): 3593-3608) mais aucun n'est réellement sélectif ou spécifique de cette kinase. Dans une grande majorité des cas, l'activité sur AXL est secondaire vis-à-vis de l'activité principale recherchée sur MET ou MER du fait de la similarité de séquence entre ces RTKs. C'est notamment le cas pour le Bosutinib ou le Cabozantinib, qui sont des inhibiteurs de kinase multi-cibles ou MTKI (*MultiTargeted Kinase Inhibitors*) déjà sur le marché, ou bien pour le BMS777607 (actuellement en phase clinique 2). C'est également le cas du dérivé de 7-azaindole NPS-1034, qui présente un profil d'inhibition relativement large, en inhibant un large panel de kinases comme AXL/DDR1/FLT3/KIT/MEK/MET/ROS1 et TIE1.

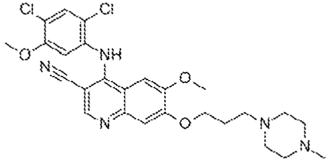

### Bosutinib

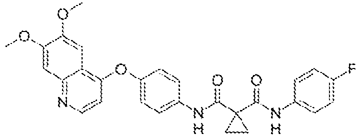

### Cabozantinib

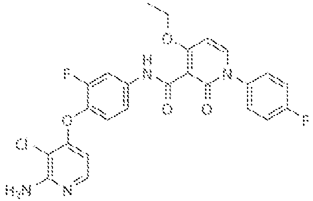

### BMS-777607

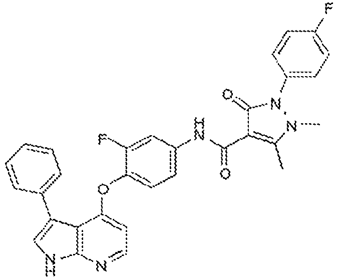

### NPS-1034

Un autre inhibiteur de la kinase AXL est le Bemcentinib(encore appelé R428 ou BGB324), actuellement en phase clinique. Ce composé - de structure bien différente des inhibiteurs de kinases actuellement sur le marché - s'est également révélé actif sur d'autres kinases comme ABL/KIT/JAK2-3/LCK/PDGFRB/TIE2. 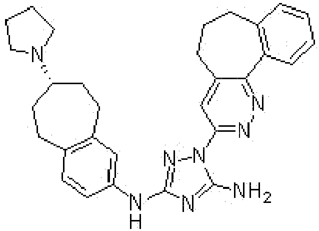 R428/BGB324

US 2003/207910 décrit des 6 aza-indoles comme composés anti-viraux. KR 2012 0061011 décrit des 7 aza-indoles comme inhibiteurs de Phospho Inositide 3 Kinase.

Il existe donc un besoin pour de nouveaux composés inhibiteurs d'AXL plus sélectifs et efficaces vis à vis des infections virales.

La présente invention propose ainsi de nouveaux dérivés de 7-azaindole inhibant fortement la kinase AXL, en vue d'une utilisation comme agents antiviraux.

De par ce profil d'inhibition bien spécifique et unique à ce jour pour ce type de structure, ces composés peuvent être utilisés en thérapie dans le traitement des infections causées par des virus utilisant le récepteur AXL pour se multiplier. Afin de combattre les infections virales, en inhibant la phosphorylation d'AXL, ces composés vont agir d'une part en bloquant l'endocytose des virus enveloppés dans leur cible cellulaire, et d'autre part en neutralisant les signaux intracellulaires contrôlés par AXL connus pour inhiber la production des interférons et des réponses antivirales de l'hôte. Les inhibiteurs de la présente invention peuvent ainsi être utilisés pour le traitement de maladies dans lesquelles AXL sont impliquées, en particulier les infections virales et notamment l'entrée virale dans les cellules.

### RESUME DE L'INVENTION

La présente invention a ainsi pour objet un composé de formule (I): 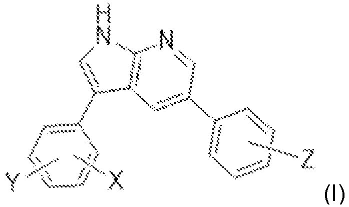 dans laquelle
X représente un atome d'hydrogène ou un atome d'halogène,
Y est choisi parmi un atome d'hydrogène, un groupe -CN, -CH₂OH, -CH=NOH, -CO₂H, un aryle optionnellement mono ou polysubstitué, un hétéroaryle optionnellement mono ou polysubstitué, et -L-(CH₂)p-W,
   - L représente -C(O)NH-, -CH₂NH-, -NHC(O)-, -CH₂N=CH-, -CH=N-, -NHC(O)NH- ou - CH₂NHC(O)-,
   - p est un entier de 0 à 2,
   - quand L représente -CH₂NH-, -NHC(O)-, -CH₂N=CH-, -CH=N-, -NHC(O)NH- ou - CH₂NHC(O)-, W est choisi parmi :
      - un atome d'hydrogène,
      - un alkyle en C₁-C₆, de préférence un méthyle,
      - un aryle en C₆-C₁₀ éventuellement mono- ou polysubstitué, avantageusement par 1 à 3 groupes indépendamment choisis parmi un atome d'halogène, un hydroxyle, un alkyle en C₁-C₆ ou un alcoxyle en C₁-C₆, et
      - un hétéroaryle à 5-10 chaînons contenant de 1 à 3 hétéroatomes indépendamment choisis parmi N, O et S, ledit hétéroaryle étant optionnellement mono- ou polysubstitué, avantageusement il est optionnellement substitué par 1 à 4 groupes indépendamment choisis parmi:
         ∘ un atome d'halogène,
         ∘ un hydroxyle,
         ∘ un groupe oxo
         ∘ un alcoxyle en C₁-C₆,
         ∘ un alkyle en C₁-C₆, dans lequel 1 ou plusieurs atomes d'hydrogène est optionnellement remplacé par un atome de fluor, et
         ∘ un aryle en C₆-C₁₀ éventuellement substitué par un atome d'halogène et/ou un alkyle en C₁-C₆;
   - quand L représente -C(O)NH-, W est choisi parmi:
      - un phényle optionnellement mono- ou polysubstitué, avantageusement il est optionnellement substitué par un atome d'halogène, un groupe hydroxyle, un alkyle en C₁-C₆, ou un groupe alcoxyle en C₁-C₆, et
      - un hétéroaryle à 5-10 chaînons comprenant de 1 à 2 hétéroatomes indépendamment choisis parmi N, O et S, ledit hétéroaryle étant optionnellement mono- ou polysubstitué, avantageusement il est optionnellement substitué par 1 à 4 substituants indépendamment choisis parmi:
         ∘ un atome d'halogène,
         ∘ un hydroxyle,
         ∘ un groupe oxo,
         ∘ un groupe alcoxyle en C₁-C₆,
         ∘ un alkyle en C₁-C₆ dans lequel 1 ou plusieurs atomes d'hydrogène est optionnellement remplacé par un atome de fluor, et
         ∘ un aryle en C₆-C₁₀ éventuellement substitué par un atome d'halogène et/ou un alkyle en C₁-C₆;
Z représente -CH₂Q ou -O(CH₂)ₘT,
   - Q représente -OH, -SO₂R₁, -NR₂R₃, ou -R₄
      - R₄ représente un alkyle en C₁-C₆,
      - R₂ et R₃ représentent chacun indépendamment un alkyle en C₁-C₆, et
      - R₄ représente un hétérocycloalkyle à 5-7 chaînons, comprenant de 1 à 2 hétéroatomes indépendamment choisis parmi N, O et S, ledit hétérocycloalkyle étant optionnellement substitué par 1 à 3 substituants indépendamment choisis parmi un alkyle en C₁-C₆, un C(O)-alkyle en C₁-C₆ et un C(O)O-alkyle en C₁-C₆,
   - m est égal à 1 ou 2,
   - T est -O(CH₂)ₙCH₃ ou -R₅
      - n est égal à 0 ou 1
      - R₅ est un hétérocycloalkyle de 5 à 7 chaînons comprenant de 1 à 2 hétéroatomes indépendamment choisis parmi N, O et S, ledit hétérocycloalkyle étant optionnellement substitué par 1 à 3 substituants indépendamment choisis parmi un alkyle en C₁-C₆, un C(O)-alkyle en C₁-C₆ et un C(O)O-alkyle en C₁-C₆;
un sel pharmaceutiquement acceptable de celui-ci ou un de leurs mélanges,
pour son utilisation dans la prévention et/ou le traitement des infections virales.

La présente invention a également pour objet une composition pharmaceutique comprenant un composé selon l'invention ou un sel pharmaceutiquement acceptable de celui-ci en tant que principe actif, et un excipient pharmaceutiquement acceptable, pour son utilisation dans la prévention ou le traitement des infections virales.

### DESCRIPTION DETAILLEE

En règle générale, les termes et définitions suivantes sont utilisés.

L'expression « couplage peptidique » dans la présente invention désigne la réaction permettant de former une liaison amide -NH-C(O)-. Les techniques utilisées dans cette réaction sont communes aux synthèses peptidiques, c'est-à-dire procèdent par activation d'un acide carboxylique pour réagir avec une amine. Les réactions de couplage peptidique utilisées dans la présente invention sont ainsi dérivées des synthèses peptidiques, et directement applicables à l'objet de la présente invention.

Les réactions de couplage peptidique sont bien connues de l'homme du métier, et peuvent notamment être réalisées en employant un agent de couplage tel que, le N,N'-dicyclohexylcarbodiimide (DCC) ou le chlorhydrate du 1-éthyl-3-(3'-diméthylaminopropyl)carbodiimide (EDC), ou le N-hydroxy-5-norbornene-2,3-dicarbodiimide), ou un benzotriazole (tel le tétrafluoroborate du O-(1H-benzotriazol-1-yl)-N,N,N',N'-tétraméthyluronium (TBTU), hexafluorophosphate de benzotriazol-1-yl-oxytris(diméthylamino)phosphonium (BOP), hexafluorophosphate du O-(7-azabenzotriazol-1-yl)-1,2,3-tétraméthyluronium (HATU), hexafluorophosphate du O-benzotriazol-1-yl-N,N,N',N'-tétraméthyluronium (HBTU), tétrafluoroborate du O-benzotriazol-1-yl-tétraméthyle (TBTU)), ou un mélange N-hydroxybenzotriazole (HOBT)/EDCI, dans un solvant tel que le chloroforme, le dichlorométhane, le dichloroéthane, l'acétate d'éthyle, le diméthylformamide (DMF), le tétrahydrofurane (THF), le diméthylsulfoxide (DMSO), la N-méthyl pyrrolidinone (NMP), de préférence à température comprise entre 20°C et 150°C.

Alternativement, un couplage peptidique a lieu par activation dans un premier temps de l'acide carboxylique par transformation en le chlorure d'acyle (notamment en présence de chlorure de thionyle ou de chlorure d'acétyle) ou un anhydride correspondant (par exemple en présence d'anhydride acétique ou isopropylique), puis réaction avec l'amine souhaitée, de préférence en présence d'une base pour neutraliser l'acide libéré lors de la réaction (notamment HCl dans le cas d'un chlorure d'acyle).

Le terme C(O) est équivalent à « C=O ».

L'expression « alkyle » ou « groupe alkyle » dans la présente invention désigne un groupe aliphatique saturé linéaire ou ramifié contenant 1 à 6 atomes de carbone, si cela n'est pas explicité. Des exemples de groupes alkyles couverts par l'objet de la présente invention sont les groupes méthyle, éthyle, propyle, butyle, tert-butyle, isopropyle.

Dans certains modes de réalisation, il est précisé qu'un ou plusieurs atomes d'hydrogène du groupe alkyle sont optionnellement remplacés par un atome de fluor. Dans ce cas, de préférence, 1 à 3 atomes d'hydrogène au plus sont concernés. Un exemple est le groupe CH₂CF₃.

L'expression "groupe aryle" ou « aryle » dans la présente invention désigne un groupe cyclique aromatique (mono- ou polycyclique) contenant entre 6 et 10 atomes de carbone. Des exemples de groupes aryles couverts par l'objet de la présente invention sont les groupes phényle, napthyle, de préférence phényle.

L'expression "groupe hétéroaryle" ou « hétéroaryle » dans la présente invention désigne un groupe cyclique aromatique (mono- ou polycyclique) à 5 à 10 chaînons, contenant entre 2 et 9 atomes de carbone et entre 1 et 3 hétéroatomes indépendamment choisis parmi l'azote, l'oxygène ou le soufre. Des exemples de groupes hétéroaryles sont les groupes furane, pyrrole, thiophène, thiazole, isothiazole, imidazole, oxazole, isoxazole, pyrazole, pyridine, pyrazine, pyridazine, pyrimidine, quinoline, indole, quinoxaline, benzofurane, dihydrobenzofurane, benzodioxole, benzotriazole, benzimidazole, de préférence choisi parmi pyrrole, imidazole, thiophène, pyridine, pyrimidine, benzofurane, benzodioxole, indole et benzimidazole.

L'expression "hétérocycloalkyle" ou « groupe hétérocycloalkyle » dans la présente invention désigne un groupe cyclique aliphatique à 5-7 chaînons, comportant un ou plusieurs (de préférence de 1 à 2) hétéroatomes indépendamment choisis parmi l'azote, l'oxygène ou le soufre, comme les morpholine, pipéridine, pipérazine, pyrrolidine, homopipérazine (1,4-diazacycloheptane). De préférence, il s'agit de la morpholine ou de la pipérazine.

L'expression "atome d'halogène" dans la présente invention désigne un atome de fluor, de chlore, de brome ou d'iode. De préférence, il s'agit du chlore ou du fluor, notamment du fluor.

L'expression "groupe alcoxyle" ou « alcoxyle » dans la présente invention désigne un groupe alkyle lié à un oxygène. Des exemples de groupes alcoxyle sont les groupes méthoxy, éthoxy, propoxy, isopropoxy, butoxy, isobutoxy, tert-butoxy. De préférence, il s'agit d'un groupe méthoxy ou éthoxy.

L'expression "groupe aryloxy" dans la présente invention désigne un groupe aryle lié à un atome d'oxygène. Des exemples de groupes aryloxy sont les groupes phényloxy.

L'expression « groupe hydroxyle » ou « hydroxyle » dans la présente invention désigne : OH.

L'expression « groupe oxo » désigne le substituant : =O.

L'expression « groupe aryle ou hétéroaryle éventuellement substitué » dans la présente invention désigne un aryle ou hétéroaryle optionnellement substitué par un ou plusieurs (de préférence 1 à 3, de manière encore préférée 1 ou 2) substituants indépendamment choisis parmi : un atome d'halogène, un groupe nitro -(NO₂), un groupe cyano (CN), un groupe alcoxyle en C₁-C₆, un groupe aryloxy en C₅-C₁₀, un groupe alkyle en C₁-C₆ dans lequel 1 ou plusieurs atomes d'hydrogène est optionnellement remplacé par un atome de fluor , un groupe hétéroaryle, un groupe hydroxyle, un groupe oxo, un groupe -CONHalkyl en C₁-C₆, un groupe -NHCOalkyl en C₁-C₆, et un groupement NR₂R₃ dans lequel R₂ et R₃ représentent indépendamment un groupe alkyle en C₁-C₆, ou un groupe aryle en C₆-C₁₀ éventuellement substitué par un atome d'halogène et/ou un groupe alkyle en C₁-C₆.

De préférence, les substituants dans l'expression « groupe aryle ou hétéroaryle éventuellement substitué » sont indépendamment choisis parmi :
un atome d'halogène, notamment un fluor ou un chlore,
un hydroxyle,
un groupe oxo,
un alcoxyle en C₁-C₆, notamment un méthoxy,
un alkyle en C₁-C₆, dans lequel 1 ou plusieurs atomes d'hydrogène est optionnellement remplacé par un atome de fluor, de préférence un groupe méthyle ou CH₂CF₃, et
un groupe aryle en C₆-C₁₀ éventuellement substitué par un atome d'halogène (notamment un fluor ou un chlore) et/ou un groupe alkyle en C₁-C₆, par exemple un fluorophényl (de préférence le 4-fluorophényle) ou un méthylfluorophényle (par exemple le 4-fluoro-2-méthylphényle);

L'expression "7-azaindole" dans la présente invention désigne le motif 1H-pyrrolo[2,3-b]pyridine :

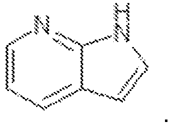

Dans la présente invention, on entend désigner par « pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

Dans la présente invention, l'expression « composition pharmaceutique » désigne toute composition consistant en une dose efficace d'au moins un composé de l'invention et au moins un excipient pharmaceutiquement acceptable. De tels excipients sont sélectionnés, en fonction de la forme pharmaceutique et de la méthode désirée d'administration, à partir des excipients usuellement connus par l'homme du métier.

On entend désigner par « sels pharmaceutiquement acceptables » d'un composé, des sels qui sont pharmaceutiquement acceptables, comme défini ici, et qui possèdent l'activité pharmacologique souhaitée du composé parent. De tels sels comprennent :
(1) les hydrates et les solvates,
(2) les sels d'addition d'acide pharmaceutiquement acceptable formés avec des acides inorganiques pharmaceutiquement acceptables tels que l'acide chlorhydrique, l'acide bromhydrique, l'acide sulfurique, l'acide nitrique, l'acide phosphorique et similaires ; ou formés avec des acides organiques pharmaceutiquement acceptables tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires, ou
(3) les sels d'addition de base pharmaceutiquement acceptable formés lorsqu'un proton acide présent dans le composé parent est soit remplacé par un ion métallique, par exemple un ion de métal alcalin, un ion de métal alcalino-terreux ou un ion d'aluminium ; soit coordonné avec une base organique ou inorganique pharmaceutiquement acceptable. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

L'expression « mélanges d'énantiomères » dans la présente invention désigne tout mélange d'énantiomères. Le mélange peut être racémique, c'est-à-dire 50/50 de chaque énantiomère en poids (w/w), ou non racémique, c'est-à-dire enrichi en l'un ou l'autre des énantiomères, par exemple de manière à obtenir un excès énantiomérique supérieur ou égal à 95%, de préférence supérieur ou égal à 98%, de manière encore préférée supérieur à 99%.

L'expression « mélanges de diastéréoisomères» dans la présente invention désigne tout mélange de diastéréomères quelle que soit la proportion.

L'expression « traitement » s'applique à tous types d'animaux, de préférence aux mammifères et plus préférentiellement aux humains. Dans le cas du traitement d'un animal non humain, l'expression référera à un traitement vétérinaire.

La présente invention concerne des composés de formule (I): 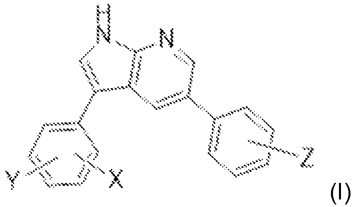 dans laquelle
X représente un atome d'hydrogène ou un atome d'halogène,
Y est choisi parmi un atome d'hydrogène, un groupe -CN, -CH₂OH, -CH=NOH, -CO₂H, un aryle optionnellement mono ou polysubstitué, un hétéroaryle optionnellement mono ou polysubstitué, et -L-(CH₂)p-W,
   - L représente -C(O)NH-, -CH₂NH-, -NHC(O)-, -CH₂N=CH-, -CH=N-, -NHC(O)NH- ou - CH₂NHC(O)-,
   - p est un entier de 0 à 2,
   - quand L représente -CH₂NH-, -NHC(O)-, -CH₂N=CH-, -CH=N-, -NHC(O)NH- ou - CH₂NHC(O)-, W est choisi parmi :
      - un atome d'hydrogène,
      - un alkyle en C₁-C₆, de préférence un méthyle,
      - un aryle en C₆-C₁₀ éventuellement mono- ou polysubstitué, avantageusement par 1 à 3 groupes indépendamment choisis parmi un atome d'halogène, un hydroxyle, un alkyle en C₁-C₆ ou un alcoxyle en C₁-C₆, et
      - un hétéroaryle à 5-10 chaînons contenant de 1 à 3 hétéroatomes indépendamment choisis parmi N, O et S, ledit hétéroaryle étant optionnellement mono- ou polysubstistué, avantageusement il est optionnellement substitué par 1 à 4 groupes indépendamment choisis parmi:
         ∘ un atome d'halogène,
         ∘ un hydroxyle,
         ∘ un groupe oxo
         ∘ un alcoxyle en C₁-C₆,
         ∘ un alkyle en C₁-C₆, dans lequel 1 ou plusieurs atomes d'hydrogène est optionnellement remplacé par un atome de fluor, et
         ∘ un aryle en C₆-C₁₀ éventuellement substitué par un atome d'halogène et/ou un alkyle en C₁-C₆;
   - quand L représente -C(O)NH-, W est choisi parmi:
      - un phényle optionnellement mono- ou polysubstitué, avantageusement il est optionnellement substitué par un atome d'halogène, un groupe hydroxyle, un alkyle en C₁-C₆, ou un groupe alcoxyle en C₁-C₆, et
      - un hétéroaryle à 5-10 chaînons comprenant de 1 à 2 hétéroatomes indépendamment choisis parmi N, O et S, ledit hétéroaryle étant optionnellement mono- ou polysubstitué, avantageusement il est optionnellement substitué par 1 à 4 substituants indépendamment choisis parmi:
         ∘ un atome d'halogène,
         ∘ un hydroxyle,
         ∘ un groupe oxo,
         ∘ un groupe alcoxyle en C₁-C₆,
         ∘ un alkyle en C₁-C₆ dans lequel 1 ou plusieurs atomes d'hydrogène est optionnellement remplacé par un atome de fluor, et
         ∘ un aryle en C₆-C₁₀ éventuellement substitué par un atome d'halogène et/ou un alkyle en C₁-C₆;
Z représente -CH₂Q ou -O(CH₂)ₘT,
   - Q représente -OH, -SO₂R₁, -NR₂R₃, ou -R₄
      - R₄ représente un alkyle en C₁-C₆,
      - R₂ et R₃ représentent chacun indépendamment un alkyle en C₁-C₆, et
      - R₄ représente un hétérocycloalkyle à 5-7 chaînons, comprenant de 1 à 2 hétéroatomes indépendamment choisis parmi N, O et S, ledit hétérocycloalkyle étant optionnellement substitué par 1 à 3 substituants indépendamment choisis parmi un alkyle en C₁-C₆, un C(O)-alkyle en C₁-C₆ et un C(O)O-alkyle en C₁-C₆,
   - m est égal à 1 ou 2,
   - T est -O(CH₂)ₙCH₃ ou -R₅
      - n est égal à 0 ou 1
      - R₅ est un hétérocycloalkyle de 5 à 7 chaînons comprenant de 1 à 2 hétéroatomes indépendamment choisis parmi N, O et S, ledit hétérocycloalkyle étant optionnellement substitué par 1 à 3 substituants indépendamment choisis parmi un alkyle en C₁-C₆, un C(O)-alkyle en C₁-C₆ et un C(O)O-alkyle en C₁-C₆;
un sel pharmaceutiquement acceptable de celui-ci ou un de leurs mélanges,
pour son utilisation dans la prévention et/ou le traitement des infections virales.

Le composé de formule (I) selon l'invention peut se trouver sous la forme d'un stéréoisomère ou d'un mélange de stéréoisomères, tels que des tautomères, énantiomères ou diastéréoisomères.

Dans un premier mode de réalisation, les composés de l'invention sont de formule (la):

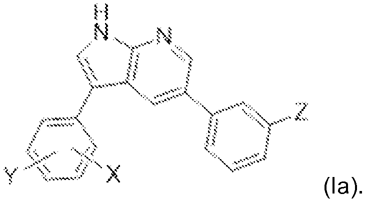

Dans un second mode de réalisation, les composés de l'invention sont de formule (Ib):

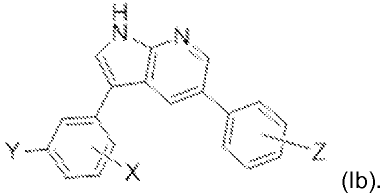

Dans un troisième mode de réalisation, les composés de l'invention sont de formule (Ic):

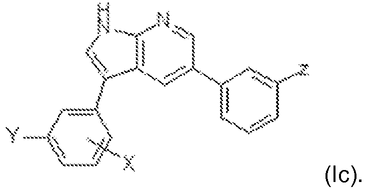

Dans un quatrième mode de réalisation, les composés de l'invention sont de formule (Id):

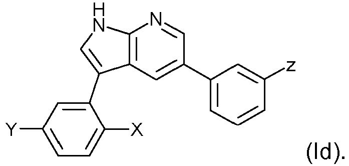

Dans les formules (la), (Ib), (Ic) et (Id), les substituants X, Y et Z sont tels que définis ci-dessus et ci-dessous.

Dans un mode de réalisation particulier, dans les composés de l'invention, Z représente - CH₂R₄ ou -O(CH₂)ₘR₅. De préférence, dans ce mode de réalisation, R₄ et R₅ représentent indépendamment un groupe pipérazine, morpholine ou homopipérazine, éventuellement substitué par un alkyle en C₁-C₆ ou un groupe C(O)O(C₁-C₆)-alkyle. Dans ce mode de réalisation, m représente de préférence 2.

Dans un mode de réalisation particulier, Z représente -CH₂OH, -CH₂SO₂CH₃, -OCH₂OCH₂CH₃, -OCH₂CH₂OCH₃, -N(CH₂CH₃)₂, 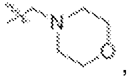 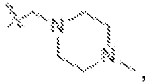 ou

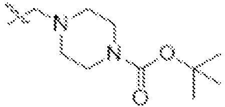

Dans un mode de réalisation préféré, Z représente -CH₂R₄ dans lequel R₄ représente un groupe pipérazine, éventuellement substitué par un alkyle en C₁-C₆, notamment un méthyle.

Dans un mode de réalisation particulier, X représente un halogène, notamment le fluor.

Dans un mode de réalisation particulier, X représente un hydrogène.

Dans un mode de réalisation particulier, Y représente -H, -CN, -CH₂OH, -CH=NOH, aryle ou hétéroaryle optionnellement mono ou polysubstitué, ou -L-(CH₂)p-W.

Dans un mode de réalisation avantageux, Y représente un aryle ou hétéroaryle optionnellement mono ou polysubstitué, ou -L-(CH₂)p-W.

Dans le cas où Y représente un aryle ou hétéroaryle optionnellement mono- ou polysubstitué, il s'agit de préférence d'un aryle optionnellement mono- ou polysubstitué, notamment d'un phényle, en particulier non substitué.

Dans un mode de réalisation encore plus avantageux, Y représente L-(CH₂)p-W. Dans le cas où Y représente -L-(CH₂)p-W, de préférence, p est alors égal à 0, 1 ou 2.

Avantageusement, lorsque Y représente L-(CH₂)p-W, L représente -C(O)NH-, -CH₂NH-, - NHC(O)-, -NHC(O)NH- ou -CH₂NHC(O)-, de préférence -CH₂NH-, -NHC(O)- ou -NHC(O)NH-

Dans le mode de réalisation où Y représente -L-(CH₂)p-W, W représente avantageusement un aryle en C₆-C₁₀ ou hétéroaryle à 5-10 chaînons contenant de 1 à 2 hétéroatomes indépendamment choisis parmi N, S et O, ledit aryle ou hétéroaryle étant optionnellement mono- ou polysubstitué.

Dans un mode de réalisation préféré, W représente un aryle en C₆-C₁₀, notamment un phényle, non substitué ou monosubstitué, de préférence par un hydroxyle.

Dans un autre mode de réalisation préféré, W représente un hétéroaryle à 5 ou 6 chainons, contenant 1 à 2 hétéroatomes indépendamment choisis parmi N, S et O, notamment N, tel que le pyrazole, la pyridine ou l'imidazole. Avantageusement ledit hétéroaryle est optionnellement substitué par 1 à 4, de préférence 1 ou 2 groupes indépendamment choisis parmi:
- un atome d'halogène, notamment un fluor,
- un groupe oxo,
- un alkyle en C₁-C₆, dans lequel 1 ou plusieurs atomes d'hydrogène est optionnellement remplacé par un atome de fluor, notamment un méthyle, et
- un aryle en C₆-C₁₀ éventuellement substitué par un atome d'halogène (notamment un fluor) et/ou un alkyle en C₁-C₆, par exemple un méthyle, un fluorophényle (de préférence le 4-fluorophényle) ou un méthylfluorophényle (par exemple le 4-fluoro-2-méthylphényle).

De préférence, W représente le pyrazole, le 3-oxo-2,3-dihydro-1H-pyrazole, la pyridine, la 2(1H)-pyridinone, la 4(1H)-pyridinone ou l'imidazole, en particulier la 2(1H)-pyridinone ou l'imidazole, optionnellement substitué par 1 à 4, de préférence 1 ou 2 groupes indépendamment choisis parmi:
- un alkyle en C₁-C₆, notamment un méthyle, et
- un aryle en C₆-C₁₀ éventuellement substitué par un alkyle en C₁-C₆, par exemple un méthyle.

En particulier, W est alors choisi parmi : 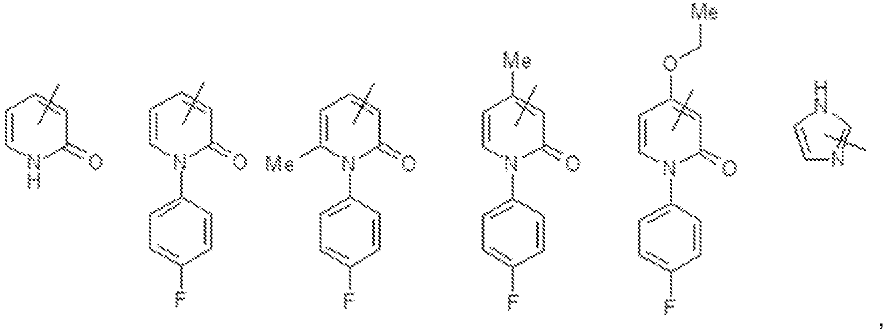 de préférence

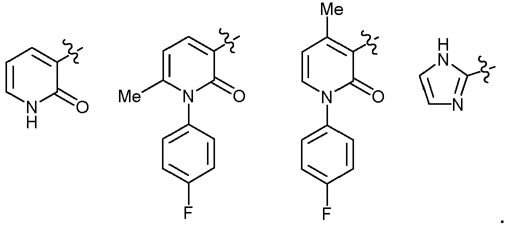

Dans un mode de réalisation avantageux, Y représente un aryle, hétéroaryle ou L-(CH₂)p-W, L représentant -CH₂NH ou -NHC(O)NH-, notamment -CH₂NH, p étant un entier de 0 à 2, et W étant choisi parmi :
un groupe phényle optionnellement substitué par un groupe hydroxyle, ou un groupe alkyle en C₁-C₆, de préférence un hydroxyle, et
un groupe hétéroaryle à 5-10 chaînons comprenant de 1 à 2 hétéroatomes indépendamment choisis parmi N, O et S, notamment N, de préférence choisi dans le groupe constitué de : pyrrole, imidazole, thiophène, pyridine, pyrimidine, benzofurane, le benzodioxole, indole et benzimidazole,

De préférence, lorsque Y représente L-(CH₂)p-W, L représentant -CH₂NH ou -NHC(O)NH-, notamment -CH₂NH, p étant un entier de 0 à 2, W est choisi parmi :
un groupe phényle optionnellement substitué par un groupe hydroxyle, ou un groupe alkyle en C₁-C₆, de préférence un hydroxyle, et
un pyrrole, imidazole, 3-oxo-2,3-dihydro-1H-pyrazole, thiophène, pyridine, 2(1H)-pyridinone, 4(1H)-pyridinone, pyrimidine, pyrimidine-2,4-dione, benzofurane, le benzodioxole, indole et benzimidazole, de manière plus préférée l'imidazole.

Dans ce mode de réalisation, Z représente de préférence -CH₂R₄ ou -O(CH₂)ₘ R₅ et de manière préférée -CH₂R₄. De préférence, dans ce mode de réalisation, R₄ et R₅ représentent indépendamment un groupe pipérazine ou morpholine, éventuellement substitué par un alkyle en C₁-C₆ ou un groupe C(O)O(C₁-C₆)-alkyle. Plus préférablement, Z représente -CH₂R₄ dans lequel R₄ représente un groupe pipérazine, substitué par un méthyle. En outre, dans ce mode de réalisation, X représente avantageusement un halogène, notamment le fluor.

Dans un autre mode de réalisation avantageux, Y représente -NHC(O)-W, avec W représentant un groupe hétéroaryle de 5 à 10 chainons, comprenant de 1 à 2 hétéroatomes choisis indépendamment parmi N, O et S, notamment N, ledit hétéroaryle étant optionnellement substitué par 1 à 4 substituants indépendamment choisis parmi :
- un groupe oxo,
- un alkyle en C₁-C₆ dans lequel 1 ou plusieurs atomes d'hydrogène est optionnellement remplacé par un atome de fluor, notamment un méthyle, et
- un aryle en C₆-C₁₀ éventuellement substitué par un atome d'halogène (notamment un fluor) et/ou un alkyle en C₁-C₆, par exemple un méthyle, un fluorophényl (de préférence le 4-fluorophényle) ou un méthylfluorophényle (par exemple le 4-fluoro-2-méthylphényle).

De préférence, dans ce mode de réalisation, le groupe hétéroaryle est choisi dans le groupe constitué de : pyrazole, pyrrole, imidazole, thiophène, pyridine, pyrimidine, benzofurane, le benzodioxole, indole et benzimidazole, optionnellement substitué par 1 à 4 substituants indépendamment choisis parmi :
- un groupe oxo,
- un alkyle en C₁-C₆, notamment un méthyle, et
- un aryle en C₆-C₁₀ éventuellement substitué par un alkyle en C₁-C₆, par exemple un méthyle.

Encore plus préférablement, dans ce mode de réalisation, W est un pyrazole, pyrrole, imidazole, 3-oxo-2,3-dihydro-1H-pyrazole, thiophène, pyridine, 2(1H)-pyridinone, 4(1H)-pyridinone, pyrimidine, pyrimidine-2,4-dione, benzofurane, le benzodioxole, indole ou benzimidazole, de manière préférée une 2(1H)- pyridinone, éventuellement substitué par 1 à 4 substituants indépendamment choisis parmi :
- un groupe alkyle en C₁-C₆, notamment un méthyle, et
- un groupe aryle en C₆-C₁₀, notamment un phényle éventuellement substitué par un atome d'halogène, notamment un fluor.

Dans ce mode de réalisation, Z représente de préférence CH₂R₄ ou O(CH₂)ₘ R₅, de manière préférée -CH₂R₄. De préférence, dans ce mode de réalisation, R₄ et R₅ représentent indépendamment un groupe pipérazine ou morpholine, éventuellement substitué par un alkyle en C₁-C₆ ou un groupe C(O)O(C₁-C₆)-alkyle. Plus préférablement, Z représente -CH₂R₄ dans lequel R₄ représente un groupe pipérazine, substitué par un méthyle.

De préférence, le composé de l'invention est choisi parmi : 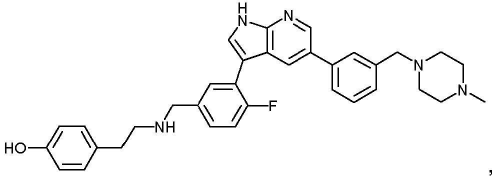 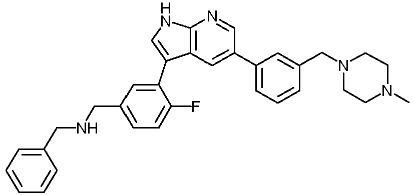 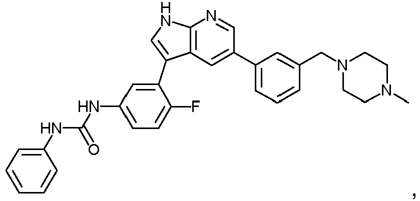 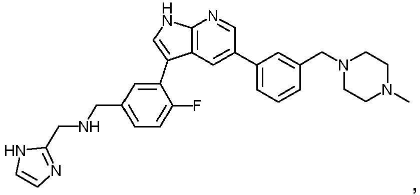 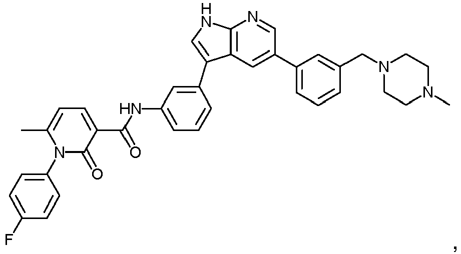 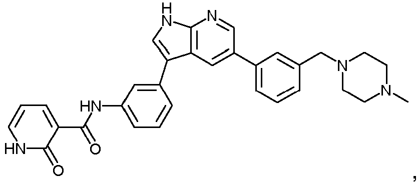 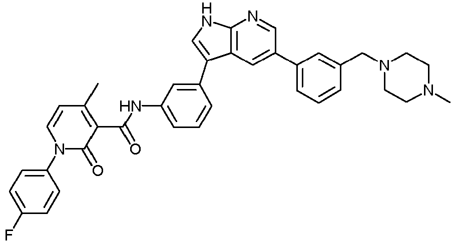 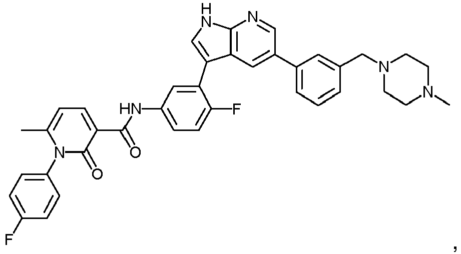 et 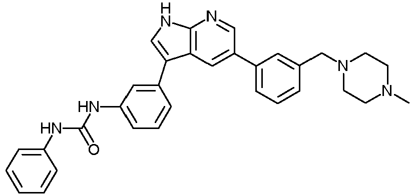 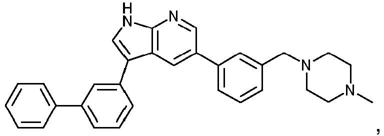 plus préférablement parmi 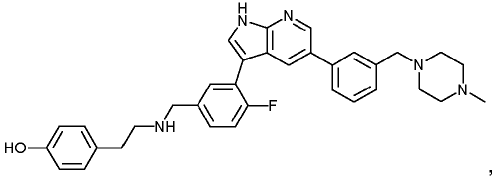 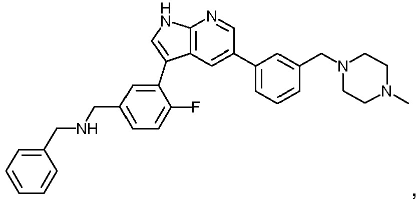 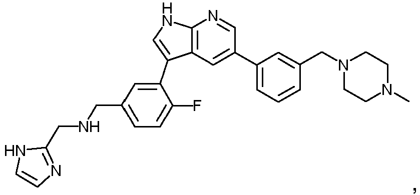 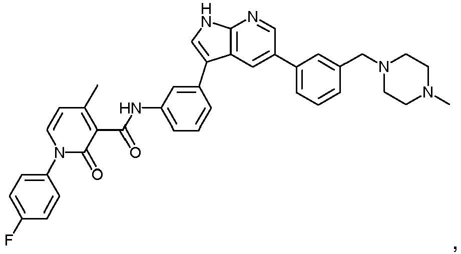 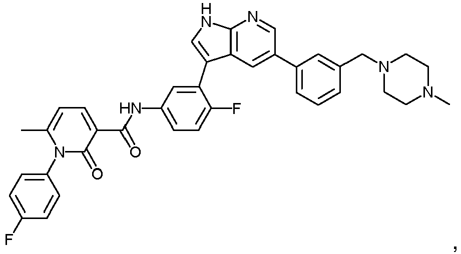 et

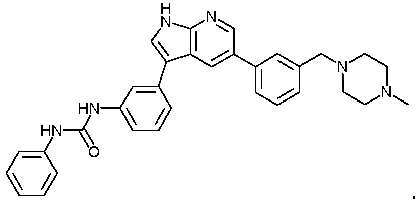

### Compositions et Applications thérapeutiques

Les composés de la présente invention inhibent les récepteurs AXL. AXL étant impliqué dans de nombreux processus biologiques et étant une cible thérapeutiquement validée, les composés de la présente invention et leurs sels pharmaceutiquement acceptables, ou les compositions de l'invention telle que définies ci-après, sont utiles en tant que médicament, notamment dans le traitement de infections virales dont le cycle infectieux dépend d'AXL et de la signalisation associée.

La présente invention porte sur des composés de formule (I) tels que définis ci-dessus pour utilisation dans la prévention et/ou le traitement des infections virales.

Plus particulièrement, les composés peuvent être employés pour préparer des compositions pharmaceutiques comprenant à titre de principe actif au moins un composé de formule (I) décrits ci-dessus ou un sel pharmaceutiquement acceptable de celui-ci, avec au moins un excipient pharmaceutiquement acceptable. Ainsi, la présente invention concerne une composition pharmaceutique comprenant un composé de formule (I) selon l'invention ou un sel pharmaceutiquement acceptable de celui-ci en tant que principe actif, et un excipient pharmaceutiquement acceptable, pour son utilisation dans la prévention et/ou le traitement des infections virales. Lesdits excipients sont choisis selon la forme pharmaceutique et le mode d'administration souhaité parmi les excipients habituels qui sont connus de l'homme du métier.

La composition pharmaceutique selon l'invention peut comprendre en outre un agent thérapeutique supplémentaire, typiquement choisi parmi un agent antiviral, un agent anti-inflammatoire, un agent immunomodulateur ou immunosuppresseur, un agent pour le traitement de troubles d'immunodéficience et d'un agent pour le traitement de la douleur. En particulier, il s'agit d'un agent utile dans un traitement contre les infections virales.

Les composés de formule (I) selon l'invention ou leurs sels pharmaceutiquement acceptables, ou la composition pharmaceutique selon l'invention sont particulièrement utiles pour la prévention et/ou le traitement des infections virales dues aux Flavivirus (dont les virus de la dengue, Zika, West Nile, Kunjin), les Alphavirus (dont les virus Chikungunya, Mayaro, Semliki Forest, Sindbis, le virus de l'encéphalite équine de l'Est, le virus de l'encéphalite equine Occidental, le virus de l'encéphalite équine du Venezuela), Les Filovirus (dont le virus Ebola, le virus de la fièvre de Marburg), les arénavirus (dont le virus de la fièvre de Lassa, le virus Junin, le virus Amapari), les picornavirus (dont virus de la stomatite vésiculeuse) , les paramyxovirus (virus influenza), les coronavirus tels que le SARS-COV2.

Dans un mode de réalisation particulier, l'invention porte sur les composés de formule (I) tels que définis ci-dessus ou la composition pharmaceutique telle que définie ci-dessus dans la prévention et/ou le traitement des infections virales due à un coronavirus, notamment le SARS-Cov2.

Dans ce mode de réalisation, le composé de formule (I) correspond préférentiellement aux composés suivants : 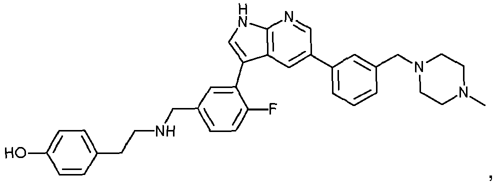 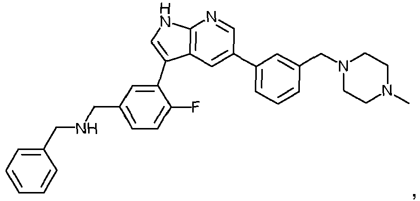 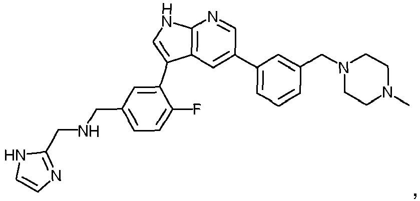 et

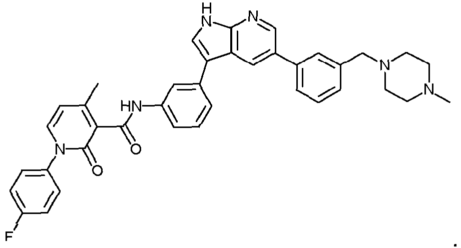

Est également décrit un kit comprenant :
a) une première composition comprenant un composé selon l'invention ou un sel pharmaceutiquement acceptable de celui-ci en tant que principe actif, et un excipient pharmaceutiquement acceptable, et
b) une deuxième composition comprenant un agent thérapeutique supplémentaire, typiquement choisi parmi un agent antiviral, un agent anti-inflammatoire, un agent immunomodulateur ou immunosuppresseur, un agent pour le traitement de troubles d'immunodéficience et d'un agent pour le traitement de la douleur, de préférence un agent utile dans un traitement contre les infections virales, en tant que produit de combinaison pour utilisation séparée, concomitante ou étalée dans le temps.

Ledit kit est utile en tant que médicament, en particulier pour la prévention et/ou le traitement des infections virales, notamment des infections virales telles que définies ci-dessus.

Les compositions pharmaceutiques selon l'invention peuvent être administrées par voie parentérale, telle que par voie intraveineuse ou intradermique, ou par voie topique, orale ou nasale.

Les formes administrables par voie parentérale incluent les suspensions aqueuses, les solutions salines isotoniques ou les solutions stériles et injectables qui peuvent contenir des agents de dispersion et/ou des mouillants pharmacologiquement compatibles. Les formes administrables par voie orale incluent les comprimés, les gélules molles ou dures, les poudres, les granules, les solutions et suspensions orales. Les formes administrables par voie nasale incluent les aérosols. Les formes administrables par voie topique incluent les patchs, les gels, les crèmes, les pommades, les lotions, les sprays, les collyres.

De préférence, les composés ou compositions de l'invention sont administrés par voie orale ou parentérale (notamment intraveineuse).

La dose efficace d'un composé de l'invention varie en fonction de nombreux paramètres tels que, par exemple, la voie d'administration choisie, le poids, l'âge, le sexe, l'état d'avancement de la pathologie à traiter et la sensibilité de l'individu à traiter.

Le traitement des pathologies ci-dessus indiquées comprend l'administration, à un patient en ayant besoin, d'une dose efficace d'un composé de formule (I) selon l'invention, ou un de ses sels pharmaceutiquement acceptable ou d'une composition selon l'invention, de préférence par voie parentérale (notamment intraveineuse) ou voie orale.

### Procédé de préparation des composés de l'invention

Sont également décrites les méthodes de préparation des composés décrits ci-dessus, notamment à partir du 5-Bromo-3-iodo-1H-pyrrolo[2,3-b]pyridine.

Une méthode est représentée dans le Schéma 1. 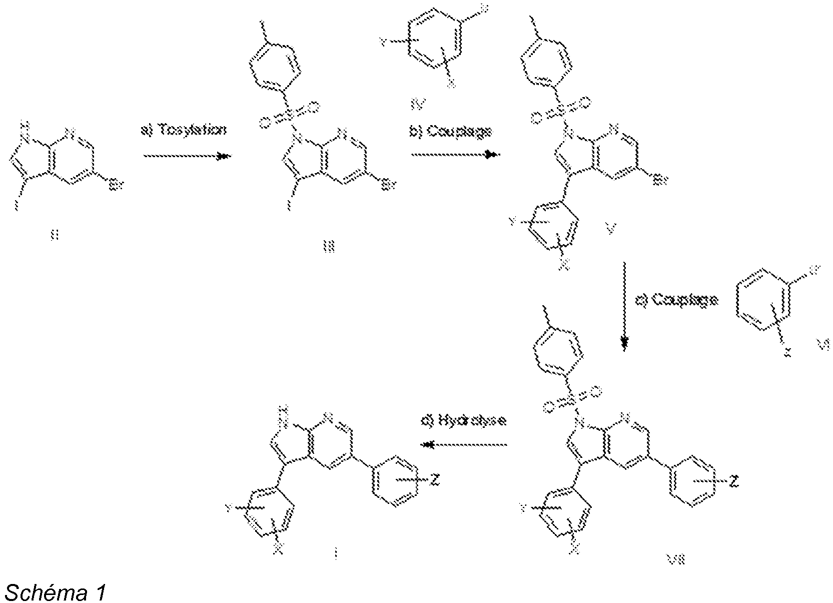 où X et Z sont tels que définis précédemment et Y représente un atome d'hydrogène, CHO, CN, CH₂OH, CO₂H, NH₂, ou un groupement phényle.

La méthode comprend alors au moins les étapes de :
a) tosylation du 5-bromo-3-iodo-1*H*-pyrrolo[2,3-b]pyridine (II), par exemple avec le chlorure de toluène-4-sulfonyle en présence d'une base comme l'hydrure de sodium, pour obtenir l'intermédiaire (III),
b) réaction de couplage de type Suzuki-Miyaura en présence d'un catalyseur au palladium comme le palladium dichloro [1,1'-Bis(diphénylphosphino)ferrocène] (Pd(dppf)Cl₂), avec l'intermédiaire de formule (IV) dans lequel U représente un acide boronique ou son ester de pinacole, pour obtenir le composé de formule (V),
c) réaction de couplage de type Suzuki-Miyaura en présence d'un catalyseur au palladium comme le palladium dichloro [1,1'-Bis(diphénylphosphino)ferrocène] (Pd(dppf)Cl₂), avec l'intermédiaire de formule (VI) dans lequel U' représente un acide boronique ou son ester de pinacole, pour obtenir l'intermédiaire de formule (VII), et
d) hydrolyse du groupement tosyle par une base, de préférence l'hydroxyde de sodium ou le carbonate de césium, pour obtenir un composé de formule (I).

La synthèse des composés intermédiaires aminés (VIII) (i.e. les composés de formule (I) pour lesquels X et Z sont tels que définis ci-dessus et Y représente un groupe (CH₂)ₙNH₂ où n est égal à 1 est représentée sur le Schéma 2. 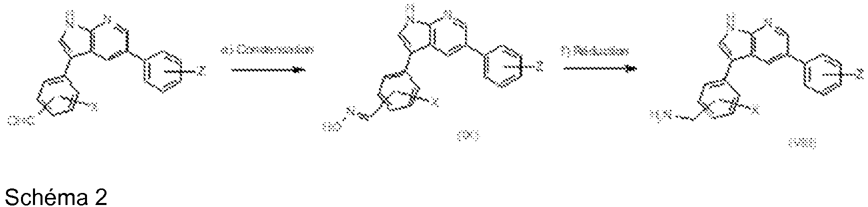 où X et Z sont tel que définis précédemment.

Dans ce mode de réalisation, la méthode de préparation des intermédiaires aminés de formule (VIII) peut comprendre au moins les étapes de :
e) condensation de l'hydroxylamine sur un composé de formule (I) dans lequel Y est CHO pour former l'oxime de formule (IX),
f) réduction de l'oxime (IX), notamment en présence de zinc sous ultra-sons pour obtenir l'intermédiaire méthylamine de formule (VIII)

L'homme du métier appliquera naturellement toutes les autres techniques de synthèse bien décrites et connues pour synthétiser ces types de composés.

Les composés de formule (I) dans lesquels Y représente un groupe L-(CH₂)ₚ-W avec L représentant un groupe amide -NHC(O)- ou -CH₂NHC(O)-, sont par exemple obtenus par une méthode de synthèse à partir des dérivés amino-7-azaindoles représentée sur le Schéma 3 : 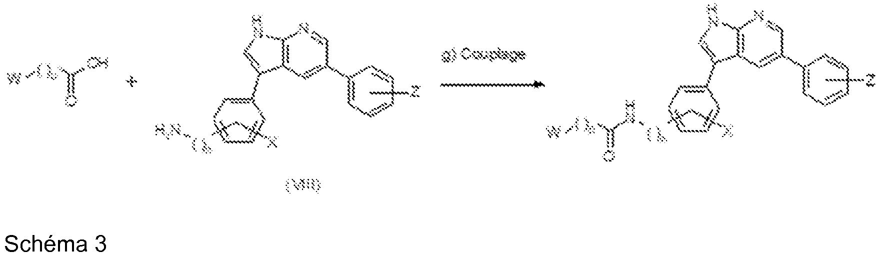 où W, X, Z et p sont tel que définis précédemment et n égal à 0 ou 1.

La méthode du Schéma 3 comprend au moins une étape de réaction de couplage peptidique entre un acide de formule W-(CH₂)ₚ-C(O)OH et l'intermédiaire aminé de formule (VIII) tel que défini précédemment, notamment en présence d'au moins un agent activateur comme l'héxafluorophosphate de 2-(7-aza-*1H*-benzotriazol-1-yl)-*N,N,N'*,*N'-*tétraméthyluronium (HATU) et d'une base comme la diisopropyléthylamine (DIEA).

L'homme du métier appliquera naturellement toutes les autres techniques de synthèse bien connues pour obtenir ces types de composés amides.

Les composés de formule (I) dans lesquels Y représente un groupe L-(CH₂)ₚ-W avec L représentant un groupe -CONH-, sont par exemple obtenus par une méthode de synthèse à partir des dérivés 7-azaindole carboxyliques de formule (X) représentée sur le Schéma 4 selon deux méthodes parmi d'autres: 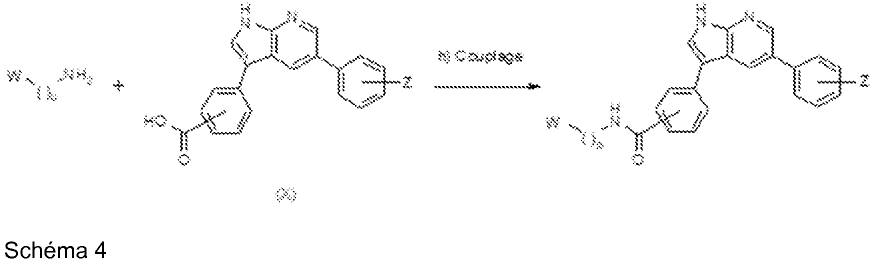 où W, Z et p sont tels que définis précédemment.

De manière avantageuse, les méthodes du Schéma 4 comprennent au moins une étape de réaction de couplage peptidique entre un acide de formule (X) et une amine de formule W-(CH₂)ₚ-NH₂, soit par couplage, soit par génération *in situ* des chlorures d'acyle par action préalable du chlorure de thionyle.

Les composés urées de la présente invention, i.e. les composés de formule (I) dans laquelle Y représente un groupe L-(CH₂)ₚ-W avec L représentant un groupe urée -NHCONH-, sont par exemple préparés selon la méthode représentée dans le Schéma 5 : 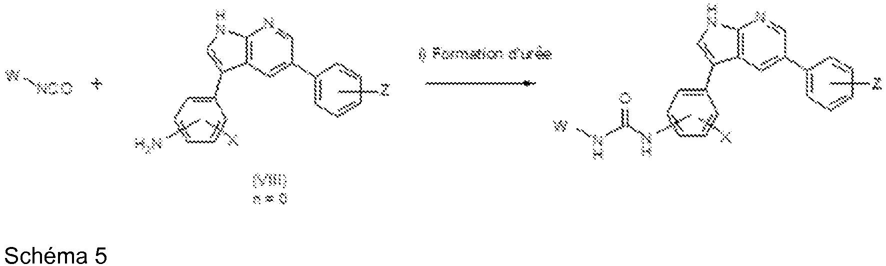 où W, X et Z sont tel que définis précédemment.

De manière avantageuse, les méthodes du Schéma 5 comprennent au moins une réaction d'un composé de formule (VIII) dans laquelle n est égal à 0, avec un isocyanate de formule W-NCO, W étant tel que défini plus haut. Lesdits isocyanates sont soit disponibles dans le commerce, soit obtenus selon des méthodes de synthèse connues de l'homme du métier. En outre, l'homme du métier appliquera naturellement toutes les autres techniques de synthèse bien connues pour obtenir ces types de composés urée.

Selon un autre mode de réalisation, concernant la méthode de synthèse des composés amines secondaires de la présente invention, i.e. les composés de formule (I) dans laquelle Y représente un groupe L-(CH₂)ₚ-W avec L représentant un groupe -CH₂NH-, deux méthodes parmi d'autres sont représentées dans le Schéma 6 : 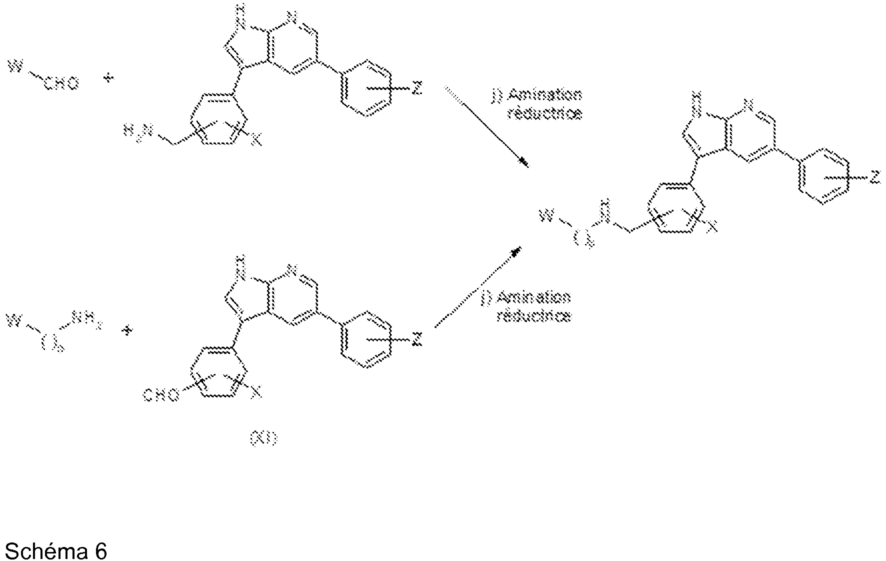 où W, X, Z et p sont tels que définis précédemment.

De manière avantageuse, les méthodes du Schéma 6 comprennent au moins une étape d'amination réductrice entre soit, un composé de formule (VIII) dans lequel n est égal à 1, avec des aldéhydes de formule W-CHO, soit entre les 7-azaindoles aldéhydes de formule (XI) et une amine de formule W(CH₂)ₚ-NH₂.

Les aldéhydes de formule W-CHO et les amines de formule W(CH₂)ₚ-NH₂ sont notamment disponibles dans le commerce, ou facilement obtenues selon des procédés de préparation connus par l'homme du métier.

L'homme du métier appliquera naturellement toutes les autres techniques de synthèse bien connues pour obtenir ces types de composés amides.

Un autre mode de réalisation concerne la méthode de synthèse des dérivés amines secondaires de formule (XII) obtenus par déprotection du composé de formule (XIII) selon le Schéma 7 suivant : 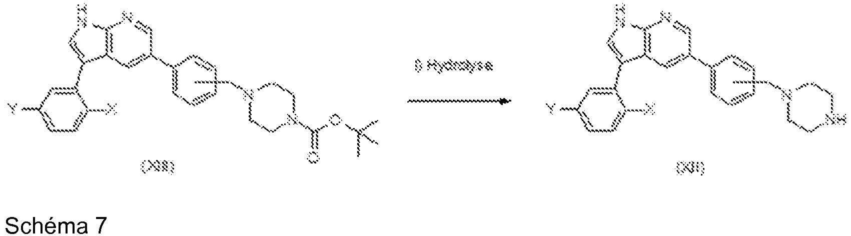 où X et Y sont tels que définis précédemment.

De manière avantageuse, la méthode comprend au moins une étape d'hydrolyse du composé carbamate de formule (XIII) en milieu acide pour former l'amine secondaire désirée de formule (XII).

L'homme du métier appliquera naturellement toutes les autres techniques de synthèse bien connues pour déprotéger l'amine secondaire attendue.

### DESCRIPTION DES FIGURES

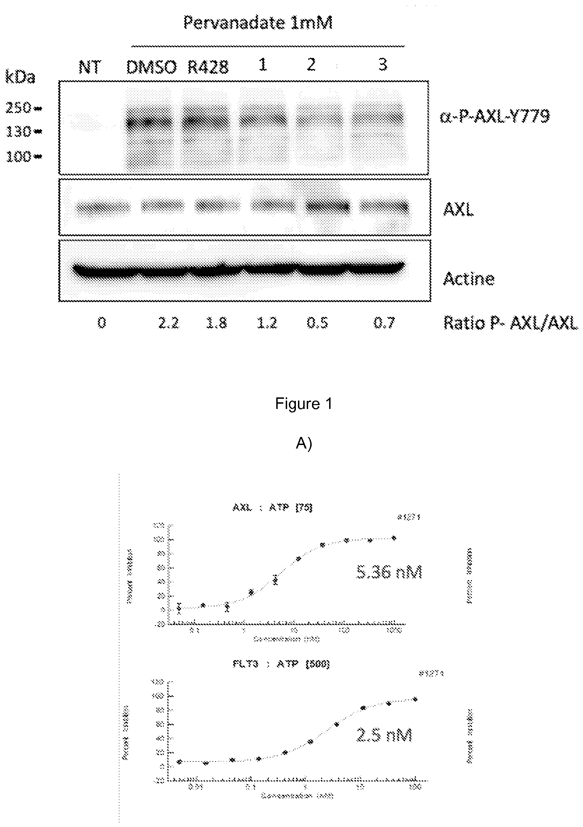

**Figure 1** : Inhibition de la phosphorylation d'Axl in cellulo par les composés 1, 2 et 3 en comparaison de R428/Bemcentinib. Les cellules A549 ont été préincubées pendant 1h avec 2.5 µM de composé ciblant Axl. Elles ont ensuite été stimulées pendant 5 min avec 1 mM de pervanadate. L'évaluation de l'inhibition de la phosphorylation du résidu Y779 du domaine intracytoplasmique de la kinase Axl se fait par immunoblot à l'aide de l'anticorps R&D Systems (AF2228) et mesure de l'intensité des signaux par densitométrie (Image J) (ratio P-Axl/Axl).

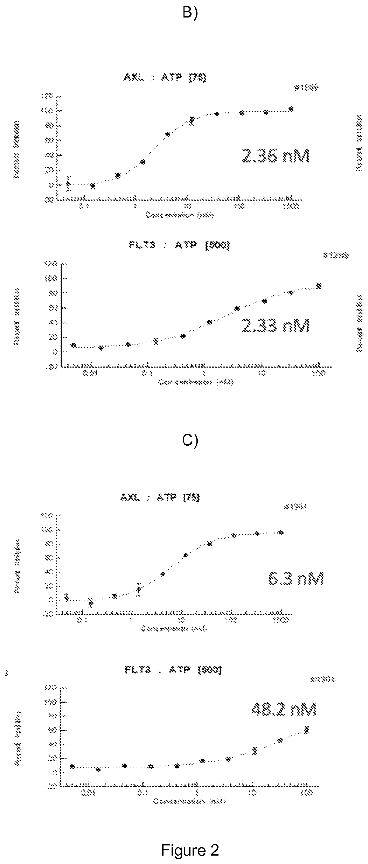

**Figure 2** : Détermination des IC50 des composés 1, 2 et 3 vis-à-vis de l'inhibition de la kinase Axl et de la kinase FLT3. 10 concentrations du composé ont été préparées par des dilutions sérielles. Les composés sont criblés dans du DMSO à 1 % final. Les kinases ont été diluées dans une concentration concentrée deux fois dans le tampon kinase. Toutes les solutions d'ATP ont été diluées dans une concentration de travail 4 fois concentrée (50 mM HEPES pH 7.5, 0.01% BRIJ-35, 10 mM MgCl2, 1 mM EGTA). Chaque composé a été incubé à 10 concentrations comprises entre 100 nM et 0,00495 nM afin de calculer l'IC₅₀. Les IC50 sont indiquées : a) résultats du composé 1 ; b) résultats du composé 2 ; c) résultats du composé 3.

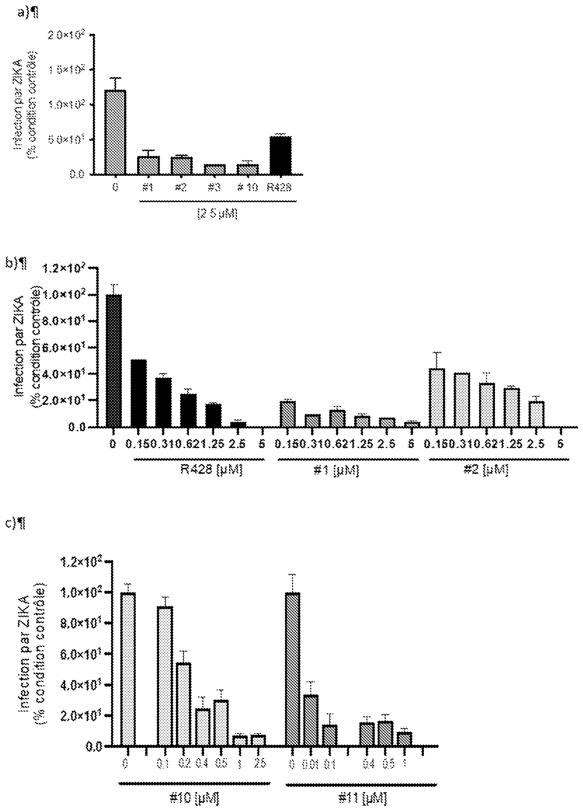

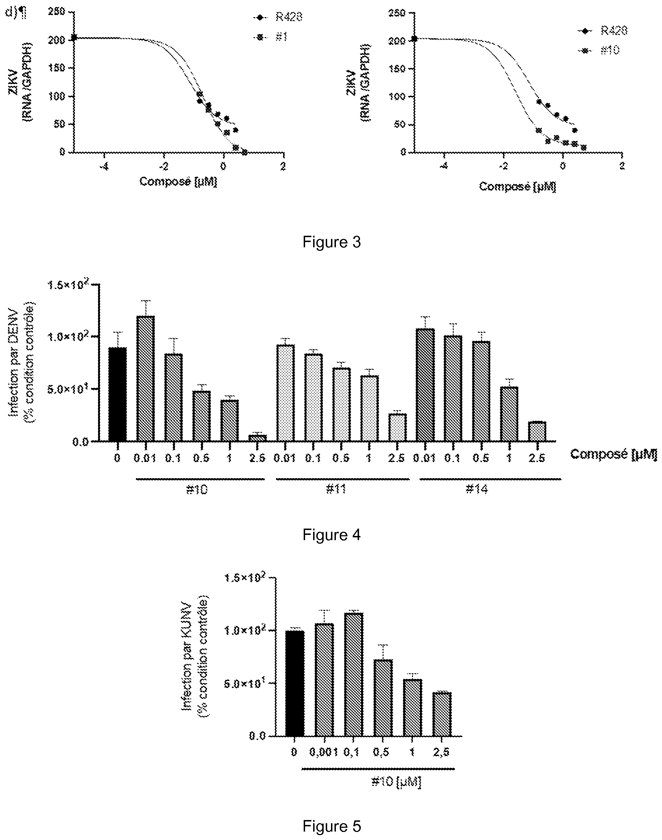

**Figure 3** **:** Activité anti-ZIKV des composés 1, 2, 3, 10 et 11 en comparaison de R428/Bemcentinib. Les cellules ont été préincubées pendant 1 heure avec les composés aux concentrations indiquées, puis infectées avec la souche virale (MOI= 0.1). Les cellules ont été maintenues en culture pendant 24 heures en présence du composé. La réplication virale résiduelle est quantifiée par qRT-PCR de l'ARN génomique viral. Les valeurs sont exprimées comme pourcentage de l'infection détectée dans la condition contrôle obtenue par incubation de cellules en présence du solvant de dilution DMSO (0). L'inhibiteur d'Axl R428/Bemcentininb est évalué en parallèle. Les valeurs sont des moyennes de triplicats + écart-type : a) résultats pour les composés 1, 2, 3, 10 et R428 à une concentration de 2,5 µM ; b) résultats pour les composés 1, 2 et R428 aux concentrations de 0,15 , 0,31, 0,62, 1,25, 2,5 et 5 µM ; c) résultats pour le composé 10 aux concentrations de 0,1, 0,2, 0,4, 0,5, 1 et 2, 5 µM et composé 11 aux concentrations de 0,001, 0,1, 0,4, 0,5, et 1 µM d) modélisation logarithmique des effets-dose obtenus pour les composés 1 (à gauche) et 10 (à droite). Les résultats obtenus avec le R428 sont présentés en parallèle.
**Figure 4** **:** Activité anti-DENV des composés 10, 11 et 14. Les cellules ont été préincubées pendant 1 heure avec les composés aux concentrations indiquées (0,01, 0,1, 0,5, 1 et 2,5 µM), puis infectées avec la souche virale DENV2(MOI= 0.1). Les cellules ont été maintenues en culture pendant 3 jours en présence du composé. La réplication virale résiduelle, mesurée par quantification des ARN viraux intracellulaires par RT-PCR, les valeurs sont exprimées comme pourcentage de l'infection détectée dans la condition contrôle obtenue par incubation de cellules en présence du solvant de dilution DMSO (0). Les valeurs sont des moyennes de triplicats + écart-type.
**Figure 5** : Activité anti-KUNV du composé 10. Les cellules ont été préincubées pendant 1 heure avec les composés aux concentrations indiquées (0,001, 0,1, 0,5, 1 et 2,5 µM), puis infectées avec la souche virale (MOI= 0.1). Les cellules ont été maintenues en culture pendant 24 heures en présence du composé. La réplication virale résiduelle est mesurée par quantification de l'activité luciférase dans le lysat cellulaire et est exprimée comme pourcentage de l'infection détectée dans la condition contrôle obtenue par incubation de cellules en présence du solvant de dilution DMSO (0). Les valeurs sont des moyennes de triplicats + écart-type.

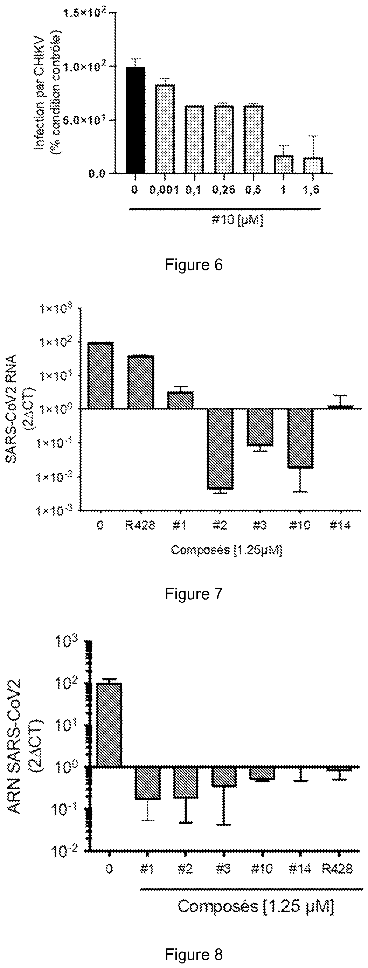

**Figure 6** **:** Activité anti-CHIKV du composé 10. Les cellules ont été préincubées pendant 1 heure avec les composés aux concentrations indiquées (0,001, 0,1, 0,25, 0,5, 1 et 1,5 µM), puis infectées avec la souche virale LR-OPY-1 (MOI= 0.1). Les cellules ont été maintenues en culture pendant 24 heures en présence du composé. La réplication virale résiduelle, mesurée par quantification de l'activité luciférase dans le lysat cellulaire, est exprimée comme pourcentage de l'infection détectée dans la condition contrôle obtenue par incubation de cellules en présence de DMSO (0). Les valeurs sont des moyennes de triplicats + écart-type.
**Figure 7** **:** Activité anti-SARS-CoV2 des composés 1, 2, 3, 10 et 14 déterminée dans la lignée VeroE6. Les cellules ont été préincubées pendant 1 heure avec les composés à une concentration de 1,25 µM, puis infectées avec la souche virale BetaCoV/France/IDF0371/2020 (MOI= 0.01). Les cellules ont été maintenues en culture pendant 24 heures en présence du composé. La réplication virale résiduelle est mesurée par quantification de l'ARN viral dans la culture cellulaire par qRT-PCR. La condition contrôle est obtenue par incubation de cellules en présence du solvant de dilution DMSO (0). Les valeurs sont des moyennes de triplicats + écart-type.
**Figure 8** : Activité anti-SARS-CoV2 des composés 1, 2, 3, 10 et 14 déterminée dans les cellules A549-ACE2. Les cellules ont été préincubées pendant 1 heure avec les composés à une concentration de 1,25 µM, puis infectées avec la souche virale BetaCoV/France/IDF0371/2020 (MOI= 0.01). Les cellules ont été maintenues en culture pendant 24 heures en présence du composé. La réplication virale résiduelle est mesurée par quantification de l'ARN viral dans la culture cellulaire par qRT-PCR. La condition contrôle est obtenue par incubation de cellules en présence du solvant de dilution DMSO (0). L'inhibiteur d'Axl R428/Bemcentininb est évalué en parallèle. Les valeurs sont des moyennes de triplicats + écart-type.

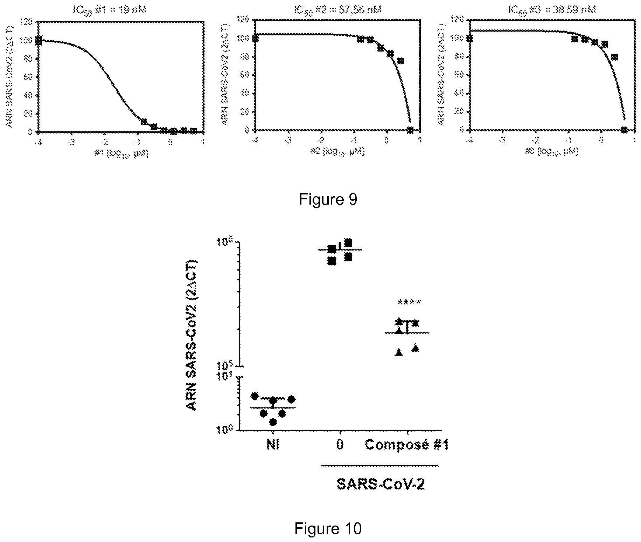

**Figure 9** : Calcul des activités spécifiques des composés 1, 2 et 3 sur le SARS-CoV2. Les cellules A549-ACE2 ont été incubées avec des concentrations croissantes de composés pendant 1h, puis infectées avec la souche virale BetaCoV/France/IDF0371/2020 (MOI= 0.01). Les cellules ont été maintenues en culture pendant 24 heures en présence du composé. La réplication virale résiduelle est mesurée par quantification de l'ARN viral dans la culture cellulaire par qRT-PCR. La condition contrôle est obtenue par incubation de cellules en présence de DMSO. Les valeurs sont des moyennes de triplicats + écart-type. Les IC50 sont calculées à l'aide du logiciel Prism (GraphPad).
**Figure 10** : Activité anti-SARS-CoV2 du composé 1 déterminée dans un modèle d'épithélium bronchique primaire humain. Les cellules ont été préincubées pendant 1 heure avec les composés utilisé à la concentration de 1,25 µM, ajouté dans le compartiment basal de la culture (MucilAir^{™}, Epithelix), Les cellules sont ensuite infectées par la souche virale BetaCoV/France/IDF0371/2020 ajoutée au compartiment apical (MOI= 0.1). Après 2 heures, l'inoculum est éliminé et les cellules sont maintenues en culture pendant 24 heures en présence du composé. La réplication virale résiduelle est mesurée par quantification de l'ARN viral dans la culture cellulaire par qRT-PCR. La condition contrôle est obtenue par incubation de cellules en présence du solvant de dilution DMSO (0). NI indique la condition correspondant aux cellules non infectées. Les valeurs individuelles ainsi que des moyennes et les écart-types sont indiqués.

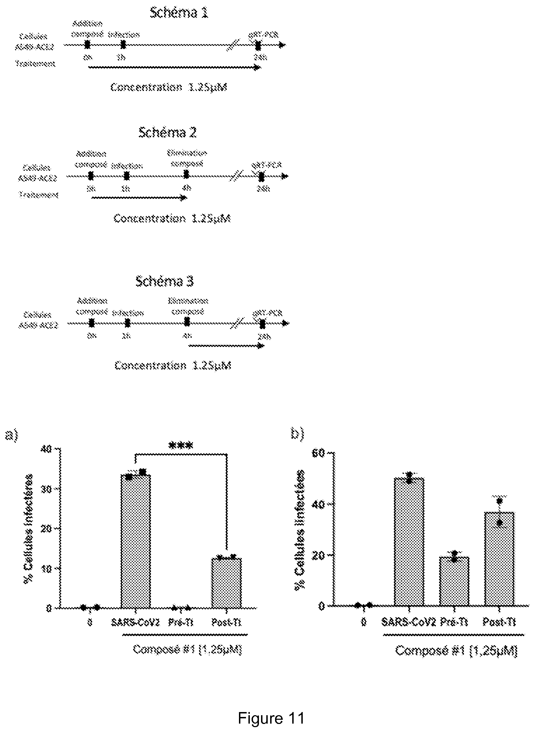

**Figure 11** : Mécanisme d'inhibition de l'infection de cellules A549-ACE2 par le composé 1. Les cellules A549-ACE2 (a) ou veroE6 (b) ont été infectées par le SARS-CoV2 (souche BetaCoV/France/IDF0371/2020) (MOI= 0.01). Le composé 1 (1.25 µM) a été ajouté à la culture cellulaire soit 1h avant le challenge viral et maintenu tout au long de l'expérience (Schéma 1, condition SARS-CoV2); 1h avant le challenge viral et seulement pendant les 4 premières heures de l'infection (Schéma 2, condition Pré-Tt) ; à partir de la 4ème heure suivant l'exposition au SARS-CoV2 (Schéma 3, condition Post-Tt). L'infection des cellules est déterminée par détection intracellulaire de la protéine Spike par FACS à l'aide d'anticorps spécifiques (anti-Spike SARS-CoV2 ; clone 1A9, Gene Tex). Les valeurs individuelles ainsi que des moyennes et les écart-types sont indiqués et comparées à la condition contrôle réalisée à partir de cellules maintenues en présence de solvant de dilution DMSO (0).

### EXEMPLES

L'invention sera mieux comprise à la lecture des exemples suivants, qui sont donnés à titre purement illustratifs et ne devraient pas être interprétés comme limitant la portée de la présente invention.

### Exemple 1 : Synthèse

### Matériel

Les synthèses et analyses ont été réalisées dans les conditions suivantes.

Résonance Magnétique Nucléaire ¹H et ¹³C:
*Appareil :* Bruker Avance 400 (400 MHz); Bruker Avance 300 (300 MHz)
*Conditions d'utilisation* : Température ambiante, déplacements chimiques exprimés en partie par million (ppm), multiplicité des signaux indiquée par des lettres minuscules (singulet s, doublet d, triplet t, quadruplet q, multiplet m), sulphoxyde de diméthyle d₆, méthanol d₄, chloroforme d₁ comme solvants deutérés.

Chromatographie Liquide Haute Pression (HPLC):
*Appareil :* Agilent Technology 1260 Infinity
*Conditions d'utilisation :* Colonne Zorbax SB-C18 ou Eclipse plus (2.1 x 50 mm), 1.8 µm; température: 30°C, débit : 0.5 mL/min pour les méthodes X et Y et 0.4 mL/min pour la méthode Z, gradient d'élution Eau (A) /Acétonitrile (B) /Acide formique 0.1% (Temps (min)/% B) :
Méthode X: 0/10, 0.3/10, 5.7/100, 6.0/100
Méthode Y: 0/10, 1/50, 6/100, 8/100
Méthode Z: 0/10, 11/100, 15/100

Spectrométrie de Masse (MS) :
*Appareil:* Quadripole Agilent Technologies 6120
*Conditions d'utilisation* : ElectroSpray (ESI) en mode positif et/ou négatif.

Pesées :
*Appareil* : Denver Instrument TP214 (précision 0.1 mg)
*Conditions d'utilisation* : Pesées effectuées au milligramme près.

Réactions sous pression :
*Appareil :* Autoclave Parr 300 mL.
*Conditions d'utilisation* : Hydrogénation sous 20 bars d'hydrogène.

Réaction sous irradiation micro-ondes :
*Appareil :* CEM Discover SP^{®}
*Conditions d'utilisation* : Puissance de 200 Watts, Vitesse d'agitation moyenne

Dans la suite, les abréviations suivantes sont utilisées :

| | | | |
|---|---|---|---|
| eq | équivalent | DMF | N,N-diméthylformamide |
| TA | Température ambiante | DMSO | diméthylsulfoxyde |

### Synthèse générale des intermédiaires 3,5-diaryl-1H-pyrrolo[2,3-b]pyridines (1)

### 1^{ère} étape, synthèse de la 5-bromo-3-iodo-1-(toluène-4-sulfonyl)-1H-pyrrolo[2,3-b]pyridine (III)

3 g de 5-bromo-3-iodo-1*H*-pyrrolo[2,3-b]pyridine (II) sont dilués dans 60 mL de THF anhydre sous argon et refroidis dans un bain de glace. 558mg (1.5 eq) d'hydrure de sodium (60%) sont ajoutés lentement et le milieu est agité à 0°C pendant 20 minutes. Puis le chlorure de tosyle (2.11g, 1,2 eq) est ajouté, et le milieu est agité à TA pendant 5 heures. Le solvant est évaporé. Le résidu obtenu est suspendu dans un minimum d'éthers de pétrole et filtré. Le précipité est lavé deux fois avec une solution d'hydroxyde de sodium 2M, puis séché sous vide pendant une nuit.

RMN ¹H (400 MHz, DMSO-d6) δ (ppm) 8.52 (d, *J* = 1.9, 1H), 8.22 (s, 1H), 8.01 (m, 3H), 7.44 (d, *J* = 8.2, 2H), 2.51 (s, 3H).

Rendement : 97% ; HPLC : 99% ; MS [M+1] : 476.9-478.9

### 2^{ème} étape, synthèse générale des 3-5-bis-aryl-1H-pyrrolo[2,3-b]pyridines (I) (dans lesquels Y = CHO, NH₂ ou un phényle substitué par un phényle)

Le 5-bromo-3-iodo-1-(toluène-4-sulfonyl)-1*H*-pyrrolo [2,3-b] pyridine (III) (100 mg),le premier acide boronique à coupler (IV) (1 eq) et le carbonate de potassium (3 eq) sont suspendus dans un mélange dioxane 3/1 eau (3 mL), et le mélange est dégazé sous Argon pendant 20 minutes. Pd(dppf)Cl₂ (2%) est ajouté et le mélange est agité à 80°C sous irradiation micro-ondes pendant 1 à 3 fois 20 minutes jusqu'à consommation totale du réactif de départ. Le milieu réactionnel est lavé par une solution aqueuse saturée en NaHCO3 et extrait à l'acétate d'éthyle. Les solvants sont évaporés, le résidu est repris dans l'acétate d'éthyle, lavé avec une solution aqueuse saturée en NaHCO3, séché sur Na2SO4 anhydre, filtré et évaporé à sec, puis purifié sur colonne de silice. Le composé est alors dissout dans un mélange dioxane/eau (3/1, 4 mL), en présence du second acide boronique ou son ester de pinacol (VI) (1,2 eq) et le carbonate de potassium (3 eq). Le milieu est dégazé à nouveau sous Argon pendant 20 minutes, Pd(dppf)Cl₂ (0.025 eq) est alors ajouté et le mélange est agité à 120°C pendant 45 minutes sous irradiation micro-ondes. 500 µL de soude 1M (3 eq) sont alors ajouté au milieu réactionnel et agité à 100°C pendant 45 minutes sous irradiation micro-ondes. Le milieu réactionnel est alors lavé avec une solution aqueuse saturée en NaHCO3, extrait à l'acétate d'éthyle, séché sur Na2SO4 anhydre, filtré et évaporé à sec, puis purifié sur colonne de silice phase normale (dichlorométhane/méthanol-ammoniaque).

| | |
|---|---|
| 4-Fluoro-3-{5-[3-(4-méthyl-pipérazin-1-ylméthyl)-phényl]-1H-pyrrolo[2,3-b]pyridin-3-yl}-benzaldéhyde | Réactifs : Premièrement l'acide 2-Fluoro-5-formylbenzèneboronique puis le 1-Méthyl-4-[3-(4,4,5,5-tétraméthyl-[1,3,2]dioxaborolan-2-yl)-benzyl]-pipérazine |
| 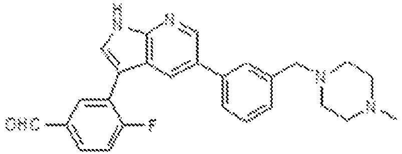 | |
| | Rendement : 95% ; HPLC : 97% ; MS [M+1] : 429.2 |
| 3-{5-[3-(4-Méthyl-pipérazin-1-ylméthyl)-phényl]-1H-pyrrolo[2,3-b]pyridin-3-yl}-phénylamine | Réactifs : Premièrement l'acide 3-Aminophénylboronique puis le 1-Méthyl-4-[3-(4,4,5,5-tétraméthyl-[1,3,2]dioxaborolan-2-yl)-benzyl]-pipérazine |
| 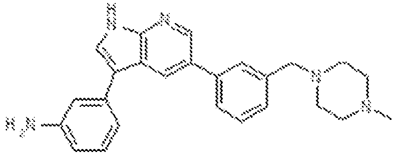 | |
| | Rendement : 66% ; HPLC : 98 % ; MS [M+1] : 398.3 |
| 3-Biphényl-3-yl-5-[3-(4-méthyl-pipérazin-1-ylméthyl)-phényl]-1H-pyrrolo[2,3-b]pyridine (Exemple 16) | Réactifs : Premièrement l'acide 3-Biphénylboronique puis le 1-Méthyl-4-[3-(4,4,5,5-tétraméthyl-[1,3,2]dioxaborolan-2-yl)-benzyl]-pipérazine |
| 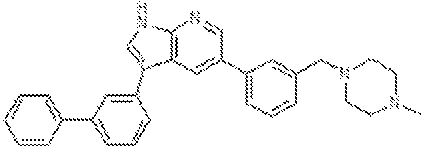 | |
| | RMN ¹H (400 MHz, DMSO-d6) δ (ppm) 12.06 (s, 1H), 8.56 (d, J = 2.0 Hz, 1H), 8.43 (d, J = 2.0 Hz, 1H), 8.06 (s, 1H), 7.98 (s, 1H), 7.83 - 7.79 (m, 1H), 7.79 - 7.75 (m, 2H), 7.67 - 7.62 (m, 2H), 7.56 (d, J = 4.9 Hz, 2H), 7.52 - 7.36 (m, 4H), 7.30 (d, J = 7.5 Hz, 1H), 3.55 (s, 2H), 2.48 - 2.20 (m, 7H), 2.13 (s, 3H). |
| | Rendement : 37% ; HPLC : 99% ; MS [M+1] : 459.3 |

### Synthèse des amines

### Méthode 1 : Synthèse de la 4-Fluoro-3-{5-[3-(4-méthyl-pipérazin-1-ylméthyl)-phényl]-1H-pyrrolo[2,3-b]pyridin-3-yl}-phénylamine

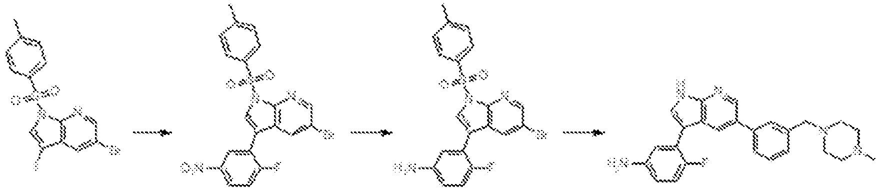

### Etape 1 : Synthèse du 5-Bromo-3-(2-fluoro-5-nitro-phényl)-1-(toluène-4-sulfonyl)-1H-pyrrolo[2,3-b]pyridine

Le 5-bromo-3-iodo-1-(toluène-4-sulfonyl)-pyrrolo [2,3-b] pyridine (460 mg), le 2-(2-Fluoro-5-nitro-phényl)-4,4,5,5-tétraméthyl-[1,3,2]dioxaborolane (1 eq) et le carbonate de potassium (3 eq) sont suspendus dans un mélange dioxane/eau (9/1, 20 mL), et dégazé sous Argon pendant 20 minutes. Pd(dppf)Cl₂ (18 mg, 0.025 eq) est ajouté et le mélange est agité pendant 2 à 4 fois 20 minutes à 50°C sous irradiation micro-ondes jusqu'à totale consommation du réactif mesurée par LC/MS. Les solvants sont évaporés, le résidu est repris dans l'acétate d'éthyle, lavé avec une solution aqueuse saturée en NaHCO3, séché sur Na2SO4 anhydre, filtré et évaporé à sec, puis purifié sur colonne de silice phase normale (éther de pétrole/dichlorométhane).

Rendement : 66% ; HPLC : 90 % ; MS [M+1] : 490.0-492.0

### Etape 2: Synthèse du 3-[5-Bromo-1-(toluène-4-sulfonyl)-1H-pyrrolo[2,3-b]pyridin-3-yl]-4-fluoro-phénylamine

Le 5-Bromo-3-(2-fluoro-5-nitro-phényl)-1-(toluène-4-sulfonyl)-1*H*-pyrrolo[2,3-b]pyridine (100 mg) est suspendu dans 3 mL d'acétate d'éthyle avec 232 mg de chlorure d'étain dihydraté (5 eq) et agité à reflux pendant 4 heures. Le milieu réactionnel est repris dans l'acétate d'éthyle et lavé avec une solution aqueuse saturée en NaHCO3. Le précipité est filtré et rincé à l'eau, puis à l'acétate d'éthyle. La phase aqueuse est extraite à l'acétate d'éthyle. Les phases organiques sont séchées sur Na2SO4 anhydre, filtrées et évaporées à sec, puis purifiées sur colonne de silice phase normale (éther de pétrole/acétate d'éthyle).

Rendement : 76% ; HPLC : 97 % ; MS [M+1] : 460.0-462.0

### Etape 3: Synthèse de la 4-Fluoro-3-{5-[3-(4-méthyl-pipérazin-1-ylméthyl)-phényl]-1H-pyrrolo[2,3-b]pyridin-3-yl}-phénylamine

Le 3-[5-Bromo-1-(toluène-4-sulfonyl)-1*H*-pyrrolo[2,3-b]pyridin-3-yl]-4-fluoro-phénylamine (475 mg), le 1-méthyl-4-[3-(4,4,5,5-tétraméthyl-[1,3,2]dioxaborolan-2-yl)-benzyl]-pipérazine (1,2 eq) et le carbonate de potassium (3 eq) sont suspendus dans un mélange dioxane/eau (9/1, 20 mL) et dégazé sous Argon pendant 20 minutes. Pd(dppf)Cl₂ (19 mg, 0.025 eq) est alors ajouté et le mélange est agité à 120°C pendant 45 minutes sous irradiation micro-ondes. 3 mL de soude 1M est alors ajouté au milieu réactionnel et agité à 100°C pendant 45 minutes sous irradiation micro-ondes. Le milieu réactionnel est alors lavé avec une solution aqueuse saturée en NaHCO3, extrait à l'acétate d'éthyle, séché sur Na2SO4 anhydre, filtré et évaporé à sec, puis purifié sur colonne de silice phase normale (dichlorométhane/méthanol ammoniacal).

Rendement : 91% ; HPLC : 98% ; MS [M+1] : 416.3

### Synthèse des différents acides carboxyliques non commerciaux :

L'acide 1-(4-Fluoro-phényl)-2-oxo-1,2-dihydro-pyridine-3-carboxylique (HPLC : 97% ; MS [M+1] : 243.1) a été obtenu conformément à la procédure décrite [Traore et al. European Journal of Medicinal Chemistry (2013), 70, 789-801].

L'acide 1-(4-Fluoro-phényl)-6-méthyl-2-oxo-1,2-dihydro-pyridine-3-carboxylique (HPLC : 97% ; MS [M+1] : 248.2) a été obtenu conformément à la procédure décrite [Flynn et al. Organic Process Research & Development (2014), 18(4), 501-510]. L'acide 1-(4-Fluoro-phényl)-4-méthyl-2-oxo-1,2-dihydro-pyridine-3-carboxylique (HPLC : 100% ; MS [M+1] : 248.2) a été obtenu comme sous-produit lors de la synthèse de l'acide 1-(4-Fluoro-phényl)-6-méthyl-2-oxo-1,2-dihydro-pyridine-3-carboxylique décrit ci-dessus.

Synthèse générale des composés amides à partir des composés amino-7-azaindole et d'acides carboxyliques.

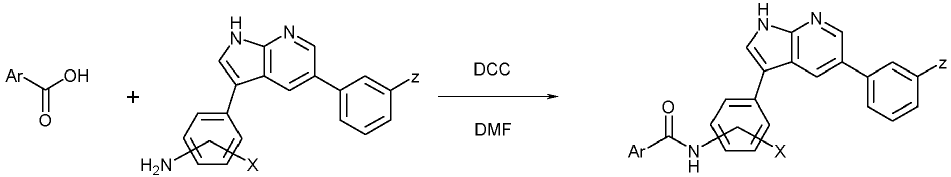

L'acide carboxylique (1eq) est dissout dans du DMF anhydre et placé dans un schlenck séché, sous atmosphère d'Argon. La N,N'-dicyclohexylcarbodiimide (1eq), puis le dérivé amino-7-azaindole (1 eq) sont ajoutés. Le milieu est alors agité à 70°C pendant une nuit. Le solvant est évaporé. Le résidu obtenu est alors dissout dans de l'acétate d'éthyle, lavé deux fois avec une solution aqueuse saturée en NaHCO3, une fois avec une solution aqueuse saturée en chlorure de sodium, séché sur Na2SO4 anhydre, filtré sur coton, évaporé à sec, et purifié sur colonne de silice phase inverse (eau/acétonitrile) avec 1% d'acide trifluoroacétique pour donner le dérivé amide désiré.

| | |
|---|---|
| 2-Hydroxy-N-(3-{5-[3-(4-méthyl-pipérazin-1-ylméthyl)-phényl]-1H-pyrrolo[2,3-b]pyridin-3-yl}-phényl)-nicotinamide (Exemple 9) | Réactifs : 3-{5-[3-(4-Méthyl-pipérazin-1-ylméthyl)-phényl]-1H-pyrrolo[2,3-b]pyridin-3-yl}-phénylamine et acide 2-Hydroxy-nicotinique |
| 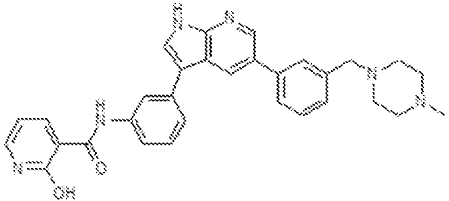 | |
| | RMN ¹H (400 MHz, DMSO-d6) δ (ppm) 13.00 - 12.55 (m, 1H), 12.27 (s, 1H), 12.04 (d, J = 2.2, 1H), 8.59 (d, J = 2.1, 1H), 8.54 - 8.48 (m, 2H), 8.36 - 8.30 (m, 1H), 7.95 (d, J = 2.6, 1H), 7.83 (dd, J = 2.2, 6.2, 1H), 7.72 - 7.63 (m, 2H), 7.57 - 7.41 (m, 4H), 7.35 - 7.28 (m, 1H), 6.59 (dd, J = 6.3, 7.2, 1H), 3.57 (s, 2H), 2.49 - 2.20 (m, 8H), 2.15 (s, 3H). Rendement : 48% ; HPLC : 99% ; MS [M+1] : 519.3 |
| 1-(4-Fluoro-phényl)-6-méthyl-2-oxo-1,2-dihydro-pyridine-3-carboxylique acide (3-{5-[3-(4-méthyl-pipérazin-1-ylméthyl)-phényl]-1H-pyrrolo[2,3-b]pyridin-3-yl}-phényl)-amide (Exemple 8) | Réactifs : 3-{5-[3-(4-Méthyl-pipérazin-1-ylméthyl)-phényl]-1H-pyrrolo[2,3-b]pyridin-3-yl}-phénylamine et acide 1-(4-Fluoro-phényl)-6-méthyl-2-oxo-1,2-dihydro-pyridine-3-carboxylique RMN ¹H (600 MHz, D₂O) δ (ppm) 8.63 (m, 1H), 8.44 (s, 1H), 7.89 (d, J = 7.0, 1H), 7.71 - 7.58 (m, 3H), 7.44 (d, J = 7.4, 1H), 7.22 - 7.08 (m, 6H), 7.08 - 7.01 (m, 2H), 7.00 - 6.92 (m, 1H), 6.35 (d, J = 7.5, 1H), 4.22 (s, 2H), 3.89 - 3.31 (m, 8H), 2.92 (s, 3H), 1.94 (s, 3H). Rendement : 67% ; HPLC : 99% ; MS [M+1] : 627.3 |
| 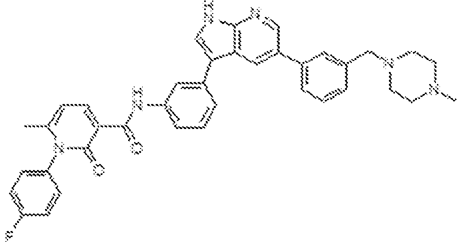 | |
| 1-(4-Fluoro-phényl)-4-méthyl-2-oxo-1,2-dihydro-pyridine-3-carboxylique acide (3-{5-[3-(4-méthyl-pipérazin-1-ylméthyl)-phényl]-1H-pyrrolo[2,3-b]pyridin-3-yl}-phényl)-amide (Exemple 10) | Réactifs : 3-{5-[3-(4-Méthyl-pipérazin-1-ylméthyl)-phényl]-1H-pyrrolo[2,3-b]pyridin-3-yl}-phénylamine et acide 1-(4-Fluoro-phényl)-4-méthyl-2-oxo-1,2-dihydro-pyridine-3-carboxylique |
| 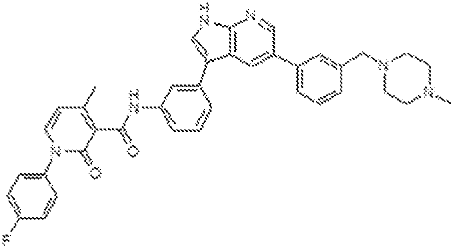 | RMN ¹H (400 MHz, DMSO-d6) δ (ppm) 12.02 (d, J = 2.5, 1H), 10.63 (s, 1H), 8.56 (d, J = 1.9, 1H), 8.50 (d, J = 1.9, 1H), 8.24 - 8.19 (m, 1H), 7.90 (d, J = 2.5, 1H), 7.71 (d, J = 7.0, 1H), 7.66 - 7.61 (m, 2H), 7.61 - 7.55 (m, 1H), 7.52 - 7.34 (m, 7H), 7.30 (d, J = 7.6, 1H), 6.36 (d, J = 7.0, 1H), 3.53 (s, 2H), 2.47 - 2.16 (m, 11H), 2.12 (s, 3H). |
| | Rendement : 54% ; HPLC : 97% ; MS [M+1] : 627.3 |
| 1-(4-Fluoro-phényl)-6-méthyl-2-oxo-1,2-dihydro-pyridine-3-carboxylique acide (4-fluoro-3-{5-[3-(4-méthyl-pipérazin-1-ylméthyl)-phényl]-1H-pyrrolo[2,3-b]pyridin-3-yl}-phényl)-amide (Exemple 11) | Réactifs : 4-Fluoro-3-{5-[3-(4-méthyl-pipérazin-1-ylméthyl)-phényl]-1H-pyrrolo[2,3-b]pyridin-3-yl}-phénylamine et acide 1-(4-Fluoro-phényl)-6-méthyl-2-oxo-1,2-dihydro-pyridine-3-carboxylique |
| 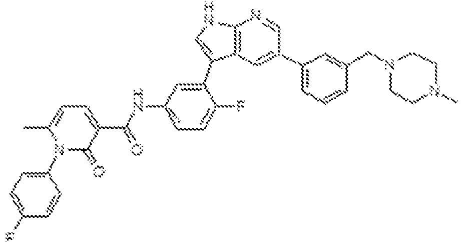 | |
| | RMN ¹H (400 MHz, DMSO-d6) δ (ppm) 12.16 (d, J = 2.2, 1H), 11.95 (s, 1H), 8.60 (d, J = 2.0, 1H), 8.52 (d, J = 7.5, 1H), 8.40 - 8.37 (m, 1H), 8.35 (dd, J = 2.6, 7.0, 1H), 7.89 - 7.85 (m, 1H), 7.70 - 7.61 (m, 2H), 7.54 - 7.40 (m, 6H), 7.35 - 7.26 (m, 2H), 6.70 (d, J = 7.9, 1H), 3.56 (s, 2H), 2.47 - 2.21 (m, 8H), 2.13 (s, 3H), 2.07 (s, 3H). |
| | Rendement : 63% ; HPLC : 99% ; MS [M+1] : 645.3 |

### Synthèse générale des urées

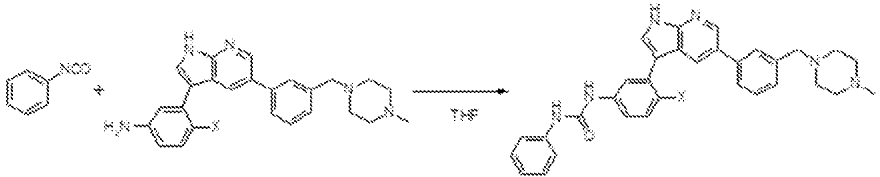

Le dérivé aminé (1 eq), l'isocyanate de phényle (1.05 eq) et la triéthylamine (2 eq) sont dissous dans du THF anhydre et agité, sous argon, à température ambiante toute une nuit. Le milieu réactionnel est quenché par une solution saturée de NaHCO3. La phase aqueuse est extraite par de l'acétate d'éthyle et séchée sur sulfate de sodium. Les phases organiques sont combinées, évaporées à sec, et purifiées sur colonne de silice phase normale (dichlorométhane/méthanol-ammoniaque) pour donner le dérivé urée désiré.

| | |
|---|---|
| 1-(3-{5-[3-(4-Méthyl-pipérazin-1-ylméthyl)-phényl]-1H-pyrrolo[2,3-b]pyridin-3-yl}-phényl)-3-phényl-urée (Exemple 14) | Réactifs : 3-{5-[3-(4-Méthyl-pipérazin-1-ylméthyl)-phényl]-1H-pyrrolo[2,3-b]pyridin-3-yl}-phénylamine et Isocyanate de phényle |
| 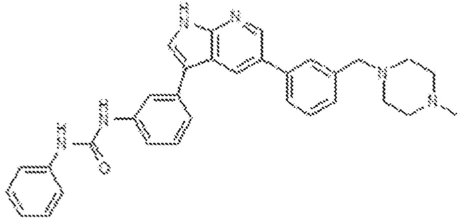 | |
| | RMN ¹H (400 MHz, DMSO-d6) δ (ppm) 12.01 (d, J = 2.0, 1H), 8.76 (bs, 1H), 8.70 (bs, 1H), 8.57 (d, J = 2.0, 1H), 8.52 (d, J = 1.9, 1H), 8.04 - 8.00 (m, 1H), 7.89 (d, J = 2.6, 1H), 7.72 - 7.62 (m, 2H), 7.53 - 7.42 (m, 3H), 7.41 - 7.24 (m, 6H), 7.01 - 6.94 (m, 1H), 3.55 (s, 2H), 2.47 - 2.19 (m, 8H), 2.12 (s, 3H). |
| | Rendement : 57% ; HPLC : 99% ; MS [M+1] : 517.3 |
| 1-(4-Fluoro-3-{5-[3-(4-méthyl-pipérazin-1-ylméthyl)-phényl]-1H-pyrrolo[2,3-b]pyridin-3-yl}-phényl)-3-phényl-urée (Exemple 3) | Réactifs : 4-Fluoro-3-{5-[3-(4-méthyl-pipérazin-1-ylméthyl)-phényl]-1H-pyrrolo[2,3-b]pyridin-3-yl}-phénylamine et Isocyanate de phényle |
| 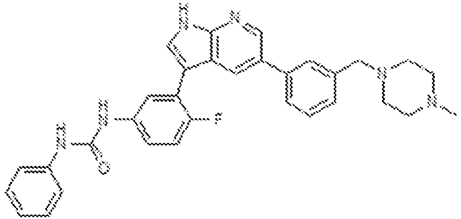 | |
| | RMN ¹H (400 MHz, DMSO-d6) δ (ppm) 12.15 (d, J = 2.1, 1H), 8.79 (bs, 1H), 8.70 (bs, 1H), 8.61 (d, J = 2.0, 1H), 8.47 - 8.42 (m, 1H), 8.06 (dd, J = 2.5, 6.9, 1H), 7.84 (t, J = 2.2, 1H), 7.72 - 7.66 (m, 1H), 7.66 - 7.63 (m, 1H), 7.52 - 7.41 (m, 3H), 7.35 - 7.21 (m, 5H), 7.01 - 6.95 (m, 1H), 3.54 (s, 2H), 2.47 - 2.17 (m, 8H), 2.12 (s, 3H). |
| | Rendement : 46% ; HPLC : 97% ; MS [M+1] : 535.3 |

### Synthèse des composés amines à partir des formyl-azaindoles

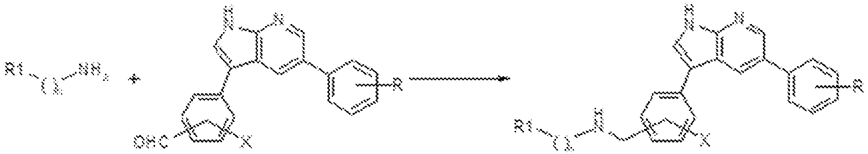

Le dérivé aldéhyde (1 eq) et le dérivé amine (1 eq) sont dissous dans un mélange méthanol / acide acétique (9/1). Le mélange est agité 3 heures à température ambiante puis le cyanoborohydrure de sodium (2 eq) est ajouté et le milieu est agité à température ambiante pour la nuit. Le solvant est évaporé, le résidu est repris en acétate d'éthyle, lavé deux fois avec une solution aqueuse saturée en NaHCO3, une fois avec une solution aqueuse saturée en chlorure de sodium, séché sur Na2SO4 anhydre, filtré sur coton et évaporé à sec. Le résidu obtenu est purifié sur colonne de silice avec un gradient de 100% dichlorométhane à 90/10 dichlorométhane/méthanol-ammoniacal.

| | |
|---|---|
| Benzyl-(4-fluoro-3-{5-[3-(4-méthyl-pipérazin-1-ylméthyl)-phényl]-1H-pyrrolo[2,3-b]pyridin-3-yl}-benzyl)-amine (Exemple 2) | Réactifs : 4-Fluoro-3-{5-[3-(4-méthyl-pipérazin-1-ylméthyl)-phényl]-1H-pyrrolo[2,3-b]pyridin-3-yl}-benzaldéhyde et Benzylamine |
| 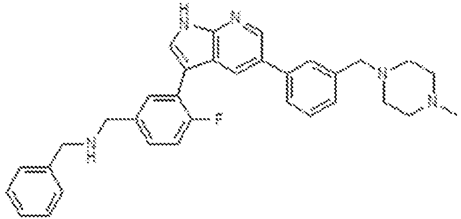 | |
| | RMN ¹H (400 MHz, DMSO-d6) δ (ppm) 12.13 (bs, 1H), 8.57 (d, J = 2.1, 1H), 8.24 (t, J = 1.9, 1H), 7.84 - 7.80 (m, 1H), 7.77 - 7.72 (m, 1H), 7.62 - 7.56 (m, 2H), 7.42 (t, J = 7.6, 1H), 7.36 - 7.15 (m, 8H), 3.74 (s, 2H), 3.72 (s, 2H), 3.51 (s, 2H), 2.47-2.15 (m, 8H), 2.13 (s, 3H). |
| | Rendement : 25% ; HPLC : 94% ; MS [M+1] : 520.3 |
| (4-Fluoro-3-{5-[3-(4-méthyl-pipérazin-1-ylméthyl)-phényl]-1H-pyrrolo[2,3-b]pyridin-3-yl}-benzyl)-(1H-imidazol-2-ylméthyl)-amine (Exemple 7) | Réactifs : 4-Fluoro-3-{5-[3-(4-méthyl-pipérazin-1-ylméthyl)-phényl]-1H-pyrrolo[2,3-b]pyridin-3-yl}-benzaldéhyde et (1H-Imidazol-2-yl)-méthylamine |
| 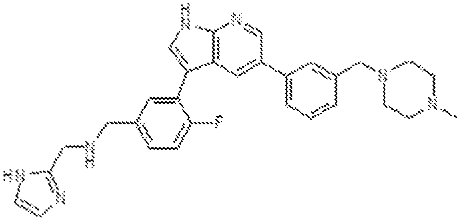 | RMN ¹H (400 MHz, DMSO-d6) δ (ppm) 12.13 (s, 1H), 8.56 (d, J = 2.1 Hz, 1H), 8.24 (s, 1H), 7.82 (s, 1H), 7.77 - 7.70 (m, 1H), 7.59 (d, J = 8.8 Hz, 2H), 7.42 (t, J = 7.5 Hz, 1H), 7.29 (dd, J = 11.3, 4.7 Hz, 3H), 6.87 (s, 2H), 3.73 (d, J = 9.6 Hz, 4H), 3.52 (s, 2H), 2.47-2.20 (m, 8H), 2.14 (s, 3H). |
| | Rendement : 46% ; HPLC : 99% ; MS [M+1] : 510.3 |
| 4-[2-(4-Fluoro-3-{5-[3-(4-méthyl-pipérazin-1-ylméthyl)-phényl]-1H-pyrrolo[2,3-b]pyridin-3-yl}-benzylamino)-éthyl]-phenol (Exemple 1) | Réactifs : 4-Fluoro-3-{5-[3-(4-méthyl-pipérazin-1-ylméthyl)-phényl]-1H-pyrrolo[2,3-b]pyridin-3-yl}-benzaldéhyde et 4-(2-Amino-éthyl)-phenol |
| 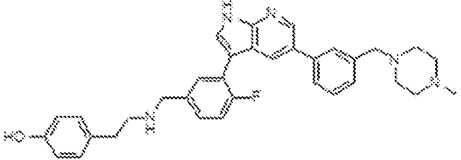 | |
| | RMN ¹H (400 MHz, DMSO-d6) δ (ppm) 12.23 - 12.01 (m, 1H), 9.08 (bs, 1H), 8.57 (d, J = 1.9, 1H), 8.27 - 8.19 (m, 1H), 7.85 - 7.78 (m, 1H), 7.76 - 7.69 (m, 1H), 7.61 (s, 2H), 7.42 (t, J = 7.6, 1H), 7.33 - 7.20 (m, 3H), 6.95 (d, J = 8.3, 2H), 6.61 (d, J = 8.3, 2H),, 3.76 (s, 2H), 3.52 (s, 2H), 2.74 - 2.65 (m, 2H), 2.65 - 2.58 (m, 2H), 2.45 - 2.19 (m, 8H), 2.13 (s, 3H).. |
| | Rendement : 25% ; HPLC : 98% ; MS [M+1] : 550.3 |

### Déprotection des dérivés aminés.

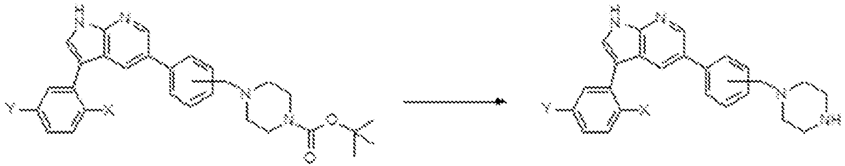

Le dérivé amine protégé (1 eq) est dissout dans du dioxane et de l'acide chlorhydrique concentré (4 eq). Le mélange est agité 2 heures à température ambiante. Le solvant est évaporé, le résidu est repris par un mélange acétate d'éthyle / eau. La phase aqueuse est neutralisée par ajout de NaHCO₃ en poudre. Le précipité est filtré, puis lavé à l'eau et séché.

### Exemple 2. TESTS BIOLOGIQUES

### A- Sélectivité des composés de l'invention vis-à-vis de la kinase AxL

Les composés de l'invention ont été évalués pour leur activité inhibitrice de la kinase Axl, de manière sélective.

### 1- Inhibition de la phosphorylation de la kinase Axl in cellulo

Les cellules A549 ont été préincubées pendant 1h avec 2.5 µM de composé 1, 2 ou 3. Elles ont ensuite été stimulées pendant 5 min avec 1 mM pervanadate, un composé connu pour activer la kinase Axl par phosphorylation.

L'inhibition de la phosphorylation du résidu Y779 du domaine intracytoplasmique d'Axl est visualisée par immunoblot à l'aide de l'anticorps R&D Systems (AF2228).

Les résultats sont illustrés en Figure 1. Ils montrent une activation bien plus faible voire nulle du résidu Y779 d'AxL en présence des composés 1, 2 et 3 par comparaison avec le contrôle (DMSO).

### 2- Inhibition sélective de la kinase Axl

L'activité inhibitrice des composés sur un panel de kinases incluant entre autres AXL et FLT3 a été évaluée par Thermo Fisher Scientific utilisant les technologie Z'-LYTE^{®} et ADAPTA ^{®} Select Screen.

L'essai biochimique basé sur la fluorescence, utilise des enzymes couplées et se base sur la différence de sensibilité des peptides phosphorylés et non phosphorylés au clivage protéolytique. Le substrat peptidique est marqué avec 2 fluorophores- un à chaque extrémité-permettant un transfert de fluorescence (FRET).

Les composés 1, 2 et 3 sont testés dans des puits à la concentration finale de 100 nM en 1% DMSO.

Les résultats sont présentés dans le tableau suivant :

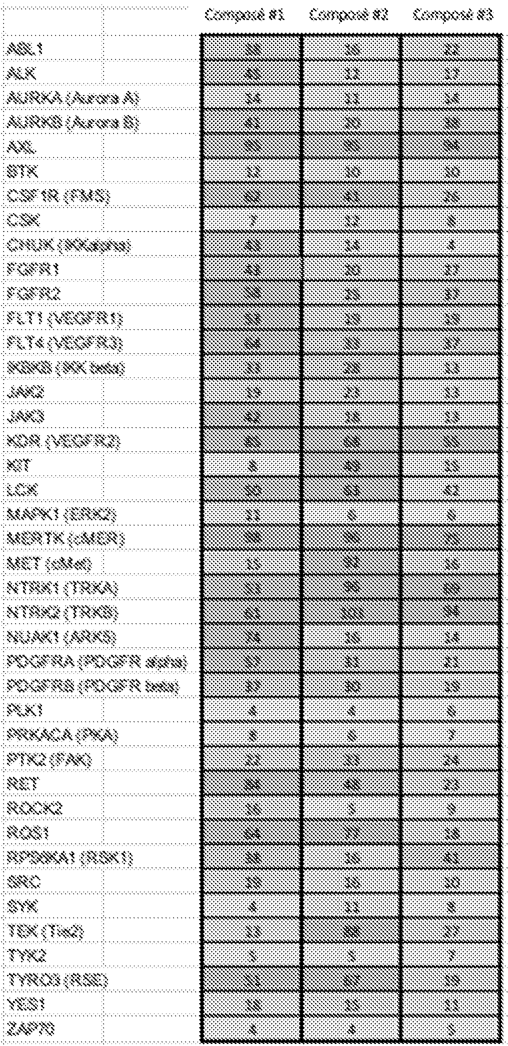

*Pourcentage d'inhibition des kinases par les composés de l'invention. Colonnes de gauche à droite : les kinases testées* - *composé 1- composé 2- composé 3.*

Les résultats montrent que chacun des composés de l'invention montre une excellente activité (95%, 95% et 94% d'inhibition) sur la kinase Axl quand vis-à-vis d'autres kinases, les résultats sont disparates d'un composé à un autre.

Pour calculer les IC₅₀ d'inhibition d'Axl et de FLT3 par les composés de l'invention, 10 concentrations de chaque composé 1, 2 et 3 ont été préparées par des dilutions sérielles au tiers à partir de la concentration de départ. Les kinases Axl ou FLT3 ont été diluées à une concentration de 2X dans le tampon kinase. Toutes les solutions d'ATP sont diluées dans une concentration de travail 4X (50 mM HEPES pH 7.5, 0.01% BRIJ-35, 10 mM MgCl2, 1 mM EGTA).

Chaque composé est incubé à 10 concentrations comprises entre 100 nM et 0,00495 nM afin de calculer l'IC₅₀.

Les résultats sont illustrés en figure 2 et résumés dans le tableau ci-dessous

| Composé | IC₅₀ Axl | IC₅₀ FLT3 |
|---|---|---|
| 1 | 5.36 nM | 2.5 nM |
| 2 | 2.36 nM | 2.33 nM |
| 3 | 6.3 nM | 48.2 nM |

Les composés 1, 2 et 3 montrent une excellente activité vis-à-vis des deux kinases Axl et FLT3. Le composé 3 montre même une sélectivité vis-à-vis d'Axl par rapport à FLT3, Axl jouant un rôle critique dans l'activation de FLT3.

### B- Evaluation anti-virale

Les composés de l'invention ont été évalués pour leur activité antivirale in vitro, en testant l'effet inhibiteur des molécules sur la réplication de virus complets infectieux. Les systèmes cellulaires choisis (cellules HeLa, A549, A549-ACE2) sont pertinents pour les modèles infectieux envisagés et ont été validés pour l'expression de la molécule Axl en cytométrie de flux.

### Modèles infectieux utilisés :

| Virus | Modèle | Souche | Cellules |
|---|---|---|---|
| ZIKV | Rapporteur mCherry | BeH-8 | HeLa |
| DENV | Rapporteur luciférase | DENV2 | HeLa |
| WNV | Rapporteur luciférase | Kunjin (KUNV) | HeLa |
| CHIKV | Rapporteur luciférase | LROPY-1 | HeLa |
| SARS-CoV2 | Isolat viral | BetaCoV/France/IDF0371/202 | A549-ACE2, VeroE6, épithelium bronchique primaire |

### 1- Activité antivirale contre le virus Zika

Les essais sont réalisés en préincubant 2.5 ×10⁴ cellules de la lignée HeLa (carcinome cervical ; ATCC# CCL-2) cultivées en plaque 96 puits avec des concentrations croissantes de composés pendant 1h à 37°C. Les cellules sont cultivées en atmosphère 5% CO2 dans un milieu Dulbecco's modified Eagle's medium (DMEM) comprenant 10% de sérum de veau et 1% de pénicilline/streptomycine. Les cellules sont ensuite exposées à la souche ZIKV BeH8 (MOI = 0.1) pendant 1h en présence du composé.

L'inoculum viral est ensuite éliminé et les cellules sont maintenues en présence des inhibiteurs pendant 24 h. L'infection des cellules est déterminée par quantification des ARN viraux détectés par qRT-PCR à l'aide du kit Luna Universal One-Step RT-PCR kit et des primers 5'-CCGCTGCCCAACACAAG et 5'- CCACTAACGTTCTTTTGCAGACATà l'aide du kit Luna Universal One-Step RT-PCR kit. Les valeurs (DCT) sont normalisées par rapport à la quantification des ARNm de la GAPDH. Les valeurs représentées sont les moyennes de triplicats + écart type.

Les conditions contrôle ont été réalisées en incubant les cellules en présence de DMSO, solvant utilisé pour la solubilisation des composés testés. Les volumes de DMSO utilisés dans ces conditions correspondent aux volumes apportés par les composés dans les conditions test.

Un traitement parallèle, dans les mêmes conditions expérimentales, a été effectué avec le R428/Bemcentinib, inhibiteurs d'Axl en phase d'essai clinique développé par la société Bergenbio.

Les résultats obtenus avec les composés 1, 2, 3, 10 et 11 sont illustrés en Figure 3. Les IC₅₀ des composés 1, 10 et du R428/Bemcetinib ont été déterminées à l'aide du logiciel Graphpad Prism.

Dans ces conditions expérimentales, les composés 1, 2, 3, 10 et 11 utilisés à une concentration supérieure ou égale à 150 nM préviennent l'infection des cellules HeLa par la Souche ZIKV BeH-8. L'efficacité in vitro de ces composés est au moins équivalente à celle du R428/Bemcentinib décrit dans la littérature pour son efficacité contre le virus ZIKV à des concentrations de 1 à 3 µM.

### 2- Activité antivirale contre le virus de la Dengue

Les essais sont réalisés en préincubant 4×10⁴ cellules de la lignée HeLa (adénocarcinome. Cervical humain ; ATCC# CCL-2) cultivées en plaque 96 puits avec des concentrations croissantes de composés pendant 1h. Les cellules sont cultivées en atmosphère 5% CO2 dans un milieu Dulbecco's modified Eagle's medium (DMEM) comprenant 10% de sérum de veau et 1% de Péniciline/streptomycine. Les cellules sont ensuite exposées à la souche DENV sous type 2 (DENV2) exprimant un gène nanoluciférase en amont de la séquence codant pour la protéine de capside, en présence des composés, pendant 1 h. L'inoculum viral est ensuite éliminé et les cellules sont maintenues en présence des inhibiteurs pendant 3 jours.

Les cellules sont lysées à l'aide du réactif Passive lysis Buffer (Promega). L'infection virale est détectée par quantification de l'activité nanoluciferase dans le lysat des cellules en présence du réactif Genofax A (Yelen) et à l'aide d'un fluorimètre Infinite F200PRO (Tecan). Les valeurs sont normalisées par rapport à la quantité de protéines totales contenues dans le lysat cellulaire et déterminées à l'aide du kit BCA Protein Assay (Pierce) par mesure de l'absorbance à 562 nm. Les valeurs représentées sont les moyennes de triplicates + écart type. Les conditions contrôle sont identiques à celles décrites précédemment.

Les résultats obtenus avec les composés 10, 11 et 14 sont illustrés en Figure 4.

Dans ces conditions expérimentales, les composés 10, 11 et 14 utilisés à une concentration supérieure ou égale à 500 nM préviennent l'infection des cellules HeLa par le sous-type DENV2.

### 3- Activité antivirale contre le virus WNV souche Kunjin

Les essais sont réalisés en préincubant 4×10⁴ cellules de la lignée HeLa (ATCC# CCL-2) cultivées en plaque 96 puits avec des concentrations croissantes de composés pendant 1h. Les cellules sont cultivées en atmosphère 5% CO2 dans un milieu Dulbecco's modified Eagle's medium (DMEM) comprenant 10% de sérum de veau et 1% de Pénicilline/streptomycine. Les cellules sont ensuite exposées au virus WNV, souche Kunjin exprimant un gène nanoluciférase en amont de la séquence codant pour la protéine de capside, en présence des composés, pendant 1 h. L'inoculum viral est ensuite éliminé et les cellules sont maintenues en présence des inhibiteurs pendant 24 h.

Les cellules sont lysées à l'aide du réactif Passive lysis Buffer (Promega). L'infection virale est détectée par quantification de l'activité nanoluciferase dans le lysat des cellules en présence du réactif Genofax A (Yelen) et à l'aide d'un fluorimètre Infinite F200PRO (Tecan). Les valeurs sont normalisées par rapport à la quantité de protéines totales contenues dans le lysat cellulaire et déterminées à l'aide du kit BCA Protein Assay (Pierce) par mesure de l'absorbance à 562 nm. Les valeurs représentées sont les moyennes de triplicates + écart type. Les conditions contrôle sont identiques à celles décrites précédemment.

Les résultats obtenus pour le composé 10 sont illustrés en Figure 5.

Dans ces conditions expérimentales, le composé 10 utilisé à une concentration supérieure ou égale à 500 nM prévient l'infection des cellules HeLa par le WNV souche Kunjin.

### 4- Activité antivirale contre le virus Chikungunya

Les essais sont réalisés en préincubant 4×10⁴ cellules de la lignée HeLa (ATCC# CCL-2) cultivées en plaque 96 puits avec des concentrations croissantes de composés pendant 1h. Les cellules sont cultivées en atmosphère 5% CO2 dans un milieu Dulbecco's modified Eagle's medium (DMEM) comprenant 10% de sérum de veau et 1% de pénicilline/streptomycine. Les cellules sont ensuite exposées au virus CHIKV, souche LR-OPY1 exprimant soit un gène luciférase, soit une séquence codant la GFP en amont de la séquence codant pour la protéine nsP3. Les cellules sont incubées pendant 1 h en présence des composés.

L'inoculum viral est ensuite éliminé et les cellules sont maintenues en présence des inhibiteurs pendant 24 h. L'infection virale est mesurée après lyse des cellules à l'aide du réactif Passive lysis Buffer (Promega), soit par quantification directe de la fluorescence de la GFP soit par quantification de l'activité luciferase dans le lysat des cellules réalisée en présence du réactif Genofax A (Yelen) et à l'aide d'un fluorimètre Infinite F200PRO (Tecan). Les valeurs sont normalisées par rapport à la quantité de protéines totales contenues dans le lysat cellulaire et déterminées à l'aide du kit BCA Protein Assay (Pierce) par mesure de l'absorbance à 562 nm. Dans les deux protocoles, les valeurs sont normalisées par rapport à la quantité de protéines totales contenues dans le lysat cellulaire et déterminées à l'aide du kit BCA Protein Assay (Pierce). Les valeurs représentées sont les moyennes de triplicates + écart type. Les conditions contrôle sont identiques à celles décrites précédemment.

Les résultats obtenus pour le composé 10 sont illustrés en Figure 6.

Dans ces conditions expérimentales, le composé 10 utilisé à une concentration supérieure ou égale à 100 nM prévient l'infection des cellules HeLa par le virus Chikungunya.

Pour chacun des modèles infectieux testés, les IC₅₀ des composés actifs ont été déterminées à l'aide du logiciel Graphpad Prism.

| Composé | Virus | | | |
|---|---|---|---|---|
| | CHIKV | KUNV | DENV | ZIKV |
| 1 | | | | 198 Nm |
| 2 | | | | 25 nM |
| 10 | 572 nM | 836 nM | 626 nM | 397 nM |
| 11 | | | > 1 µM | < 100 nM |
| 14 | | > 1 µM | > 1 µM | |
| R428/Bemcentinib (référence) | 239 nM | ND | 488 nM | 285 nM |

Ces évaluations indiquent que plusieurs composés présentent une EC₅₀ inférieure à 1µM.

### 5- Activité antivirale contre le coronavirus SARS-CoV2

L'activité inhibitrice des composés de l'invention sur l'infection par le SARS-CoV2 a été évaluée in vitro par infection de trois lignées pertinentes pour l'étude de ce pathogène : la lignée de singe vert VeroE6 (épithélium immortalisé de rein, ATCC#CRL-1586), la lignée A549-ACE2 produite par transfection du récepteur ACE2 dans la lignée A549 (carcinome épithélial pulmonaire ; ATCC# CCL-185), des cellulaires d'épithelium bronchique primaire commerciales (Mucilair Epithélix).

### a- Évaluation des activités antivirales en cellules VeroE6 et A549-ACE2

Les tests d'infection sont réalisés en préincubant 4×10⁴ cellules VeroE6 ou A549-ACE2 cultivées en plaque 96 puits avec les concentrations indiquées de composés pendant 1h. Les cellules sont cultivées en atmosphère 5% CO2 dans un milieu Dulbecco's modified Eagle's medium (DMEM) comprenant 2% de sérum de veau et 1% de pénicilline/streptomycine. Les cellules ont ensuite été exposées au virus SARS-CoV2, souche BetaCoV/France/IDF0371/2020 (MOI = 0.01) pendant 2h en présence des composés avant élimination de l'innoculum, lavage et remise en culture des cellules en présence des composés. 24h après le challenge viral, l'infection est quantifiée par amplification des ARN viraux par qRT-PCR à l'aide du kit Luna Universal One-Step RT-PCR kit. Les valeurs (DCT) sont normalisées par aux ARNm de la GAPDH. Les valeurs représentées sont les moyennes de triplicates + écart type. Les conditions contrôle ont été réalisées en incubant les cellules en présence de DMSO, solvant utilisé pour la solubilisation des composés testés. Les volumes de DMSO utilisés dans ces conditions correspondent aux volumes apportés par les composés dans les conditions test.

Un traitement parallèle, dans les mêmes conditions expérimentales, a été effectué avec le R428/Bemcentinib, inhibiteurs d'Axl en phase d'essai clinique développé par la société Bergenbio.

Les résultats obtenus pour les composés 1, 2, 3, 10 et 14 sont illustrés en Figures 7 (lignée VeroE6) et 8 (lignée A549-ACE2).

Dans ces conditions expérimentales, les composés 1, 2, 3, 10 et 14 préviennent l'infection des cellules VeroE6 et A549-ACE2 par le virus SARS-CoV2.

Les IC₅₀ des composés 1, 2 et 3 ont été déterminées sur les cellules VeroE6 et A549-ACE2 à l'aide du logiciel Graphpad Prism.

Les résultats obtenus sont illustrés en Figure 9 et dans le tableau suivant :

| | IC₅₀ | | |
|---|---|---|---|
| Composé | 1 | 2 | 3 |
| A549-ACE2 | 19 nM | 57 nM | 38 nM |
| Vero E6 | 77 nM | 289 nM | ND |

*Les composés 1, 2, 3, 10 et 14 présentent une activité anti-SARS-CoV2 in vitro.*

### b- Évaluation des activités antivirales en modèle d'épithélium bronchique humains primaires

Les tests d'infection des épithélium bronchiques primaires ont été réalisés à partir du modèle MucilAir^{™} Human airway epithelial cells (Epithelix) cultivé en milieu approprié (milieu HAE, Epithelix). Les composés sont ajoutés au compartiment basal de la culture et préincubés pendant 1h à 37°C. L'inoculum viral (MOI= 0.01) est ensuite ajouté au compartiment apical en même temps que les composés. Après 2h à 37°C, l'inoculum viral est éliminé, remplacé par du milieu contenant le composé à tester et la culture est maintenue à 37°C pendant 24 heures. L'infection des cellules est déterminée par quantification des ARN viraux détectés par qRT-PCR à l'aide du kit Luna Universal One-Step RT-PCR kit. Les valeurs (DCT) sont normalisées par rapport aux mARN de la GAPDH.

Les résultats obtenus pour le composé 1 sont illustrés en Figure 10.

Ces évaluations indiquent que le composé 1 inhibe l'infection de l'épithélium bronchique primaire par le SARS-CoV2.7- Activité préventive et curative du composé #1 contre le coronavirus SARS-CoV2
L'activité inhibitrice du composé 1 sur l'infection par le SARS-CoV2 a été évaluée in vitro dans le cadre d'un traitement préventif (Pré-Tt) ou curatif (Post-Tt) de la lignée A549-ACE2 produite par transfection du récepteur ACE2 dans la lignée A549 (carcinome épithélial pulmonaire ; ATCC# CCL-185) a), et de la lignée de singe vert VeroE6 (épithélium immortalisé de rein, ATCC#CRL-1586).

Les tests d'infection sont réalisés sur 4×10⁴ cellules cultivées en plaque 96 puits, en atmosphère 5% CO2 dans un milieu Dulbecco's modified Eagle's medium (DMEM) comprenant 2% de sérum de veau et 1% de pénicilline/streptomycine. Les cellules sont exposées au virus SARS-CoV2, souche BetaCoV/France/IDF0371/2020 (MOI = 0.01). Les composés sont ajoutés aux cellules soit 1h avant l'infection et maintenus pendant 24h ; soit ajoutés aux cellules 1h avant l'infection et éliminés par lavage 4h après le challenge viral ; soit ajoutés aux cellules 4h après l'exposition au virus et maintenus pendant 24 h (Post-Tt). L'infection résiduelle est quantifiée par amplification des ARN viraux par qRT-PCR à l'aide du kit Luna Universal One-Step RT-PCR kit. Les valeurs (DCT) sont normalisées par rapport aux ARNm de la GAPDH.

Les conditions contrôle ont été réalisées en incubant les cellules en présence de DMSO (SARS-CoV2), solvant utilisé pour la solubilisation des composés testés. Les volumes de DMSO utilisés dans ces conditions correspondent aux volumes apportés par les composés dans les conditions test.

Les résultats obtenus sont illustrés en Figure 11.

Ces évaluations indiquent que l'utilisation préventive des composés inhibe l'infection par SARS-CoV2 dans les cellules humaines A549-ACE2 et les cellules de singe VeroE6. L'administration des composés après le challenge viral réduit la multiplication virale dans les cellules A549-ACE2, mais pas dans la lignée VeroE6 qui se caractérise par un défaut de production des interférons de type I.

En conclusion, les composés actifs sur les différents modèles infectieux testés sont les suivantes :

| Composé | Virus | | | | | |
|---|---|---|---|---|---|---|
| | CHIKV | MAYV | KUNV | DENV | ZIKV | SARS-CoV2 |
| 1 | ND | ND | ACTIF | ND | ACTIF | ACTIF |
| 2 | ND | ND | ACTIF | ND | ACTIF | ACTIF |
| 3 | ND | ND | ND | ND | ND | ACTIF |
| 10 | ACTIF | ACTIF | ACTIF | ACTIF | ACTIF | ACTIF |
| 11 | ND | ND | ND | ACTIF | ACTIF | ND |
| 14 | ND | ND | ACTIF | ACTIF | ND | ND |

## Revendications

1. Composé de formule (I): 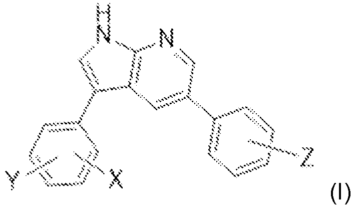 dans laquelle
X représente un atome d'hydrogène ou un atome d'halogène,
Y est choisi parmi un atome d'hydrogène, un groupe -CN, -CH₂OH, -CH=NOH, -CO₂H, un aryle optionnellement mono ou polysubstitué, un hétéroaryle optionnellement mono ou polysubstitué, et -L-(CH₂)p-W,
- L représente -C(O)NH-, -CH₂NH-, -NHC(O)-, -CH₂N=CH-, -CH=N-, -NHC(O)NH- ou - CH₂NHC(O)-,
- p est un entier de 0 à 2,
- quand L représente -CH₂NH-, -NHC(O)-, -CH₂N=CH-, -CH=N-, -NHC(O)NH- ou - CH₂NHC(O)-, W est choisi parmi :
• un atome d'hydrogène,
• un alkyle en C₁-C₆, de préférence un méthyle,
• un aryle en C₆-C₁₀ éventuellement mono- ou polysubstitué, avantageusement par 1 à 3 groupes indépendamment choisis parmi un atome d'halogène, un hydroxyle, un alkyle en C₁-C₆ ou un alcoxyle en C₁-C₆, et
• un hétéroaryle à 5-10 chaînons contenant de 1 à 3 hétéroatomes indépendamment choisis parmi N, O et S, ledit hétéroaryle étant optionnellement mono- ou polysubstistué, avantageusement il est optionnellement substitué par 1 à 4 groupes indépendamment choisis parmi
∘ un atome d'halogène,
∘ un hydroxyle,
∘ un groupe oxo,
∘ un alcoxyle en C₁-C₆,
∘ un alkyle en C₁-C₆ dans lequel 1 ou plusieurs atomes d'hydrogène est optionnellement remplacé par un atome de fluor, et
∘ un aryle en C₆-C₁₀ éventuellement substitué par un atome d'halogène et/ou un alkyle en C₁-C₆;
- quand L représente -C(O)NH-, W est choisi parmi:
• un phényle optionnellement mono- ou polysubstitué, avantageusement il est optionnellement substitué par un atome d'halogène, un groupe hydroxyle, un alkyle en C₁-C₆, ou un groupe alcoxyle en C₁-C₆, et
• un hétéroaryle à 5-10 chaînons comprenant de 1 à 2 hétéroatomes indépendamment choisis parmi N, O et S, ledit hétéroaryle étant optionnellement mono- ou polysubstitué, avantageusement il est optionnellement substitué par 1 à 4 substituants indépendamment choisis parmi
∘ un atome d'halogène,
∘ un hydroxyle,
∘ un groupe oxo,
∘ un groupe alcoxyle en C₁-C₆,
∘ un alkyle en C₁-C₆ dans lequel 1 ou plusieurs atomes d'hydrogène est optionnellement remplacé par un atome de fluor, et
∘ un aryle en C₆-C₁₀ éventuellement substitué par un atome d'halogène et/ou un alkyle en C₁-C₆;
Z représente -CH₂Q ou -O(CH₂)ₘT,
- Q représente -OH, -SO₂R₁, -NR₂R₃, ou -R₄
- R₁ représente un alkyle en C₁-C₆,
- R₂ et R₃ représentent chacun indépendamment un alkyle en C₁-C₆, et
- R₄ représente un hétérocycloalkyle à 5-7 chaînons, comprenant de 1 à 2 hétéroatomes indépendamment choisis parmi N, O et S, ledit hétérocycloalkyle étant optionnellement substitué par 1 à 3 substituants indépendamment choisis parmi un alkyle en C₁-C₆, un C(O)-alkyle en C₁-C₆ et un C(O)O-alkyle en C₁-C₆,
- m est égal à 1 ou 2,
- T est -O(CH₂)ₙCH₃ ou -R₅
• n est égal à 0 ou 1
• R₅ est un hétérocycloalkyle de 5 à 7 chaînons comprenant de 1 à 2 hétéroatomes indépendamment choisis parmi N, O et S, ledit hétérocycloalkyle étant optionnellement substitué par 1 à 3 substituants indépendamment choisis parmi un alkyle en C₁-C₆, un C(O)-alkyle en C₁-C₆ et un C(O)O-alkyle en C₁-C₆;
ou un sel pharmaceutiquement acceptable de celui-ci ou un de leurs mélanges,
pour son utilisation dans la prévention et/ou le traitement des infections virales.

2. Composé pour son utilisation selon la revendication 1, de formule (Id) : 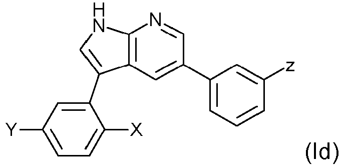 dans laquelle X, Y et Z sont tels que définis dans la revendication 1.

3. Composé pour son utilisation selon la revendication 1 ou 2, **caractérisé en ce que** Z représente -CH₂R₄ dans lequel R₄ représente un groupe pipérazine, éventuellement substitué par un alkyle en C₁-C₆, notamment un méthyle.

4. Composé pour son utilisation selon l'une quelconque des revendications 1 à 3, **caractérisé en ce que** X représente un halogène, notamment le fluor.

5. Composé pour son utilisation selon l'une quelconque des revendications 1 à 4, **caractérisé en ce que** Y représente un aryle, hétéroaryle ou L-(CH₂)ₚ-W, L représentant -CH₂NH ou - NHC(O)NH-, p étant un entier de 0 à 2, et W étant choisi parmi :
- un phényle optionnellement substitué par un groupe hydroxyle, ou un groupe alkyle en C₁-C₆, et
- un hétéroaryle à 5-10 chaînons comprenant de 1 à 2 hétéroatomes indépendamment choisis parmi N, O ou S.

6. Composé pour son utilisation selon la revendication 5, **caractérisé en ce que** Y représente L-(CH₂)ₚ-W, L représentant -CH₂NH ou -NHC(O)NH-, p étant un entier de 0 à 2, et W étant choisi parmi :
- un phényle optionnellement substitué par un groupe hydroxyle, et
- un imidazole.

7. Composé pour son utilisation selon l'une quelconque des revendications 1 à 4, **caractérisé en ce que** Y représente -NHC(O)-W, avec W représentant un groupe hétéroaryle de 5 à 10 chainons, comprenant de 1 à 2 hétéroatomes indépendamment choisis parmi N, O ou S, ledit hétéroaryle étant optionnellement substitué par 1 à 4 substituants indépendamment choisis parmi :
- un groupe oxo,
- un groupe alkyle en C₁-C₆ dans lequel 1 ou plusieurs atomes d'hydrogène est optionnellement remplacé par un atome de fluor, et
- un groupe aryle en C₆-C₁₀ éventuellement substitué par un atome d'halogène et/ou un groupe alkyle en C₁-C₆.

8. Composé pour son utilisation selon la revendication 7, **caractérisé en ce que** W est une 2(1H)- pyridinone, éventuellement substituée par 1 à 4 substituants indépendamment choisis parmi :
- un méthyle, et
- un phényle éventuellement substitué par un atome de fluor.

9. Composé pour son utilisation selon la revendication 1, choisi parmi : 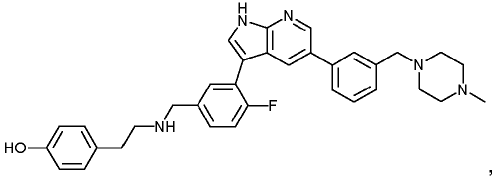 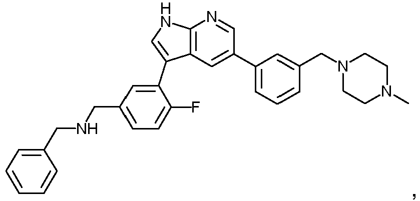 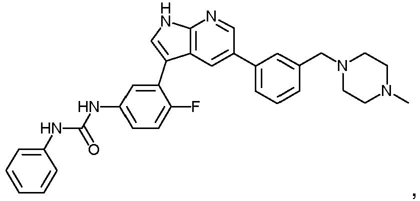 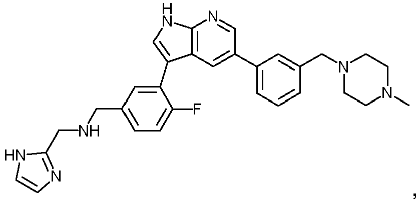 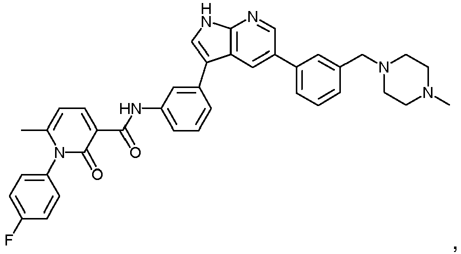 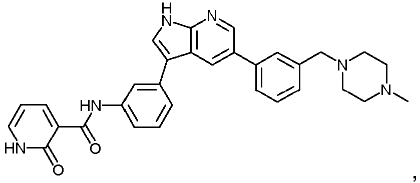 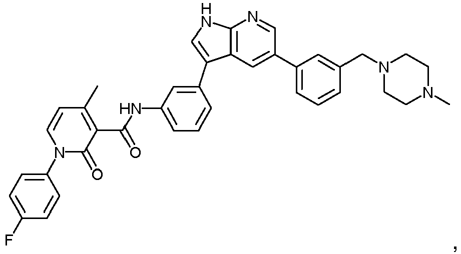 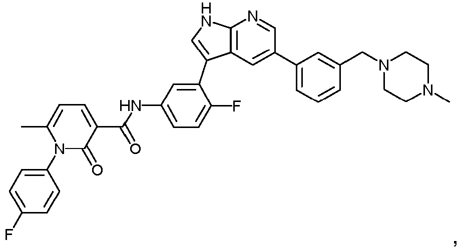 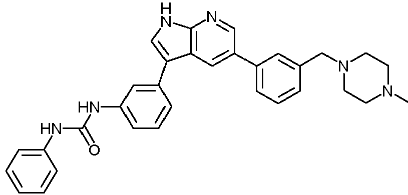 et 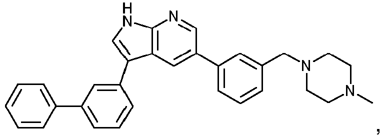 préférablement parmi 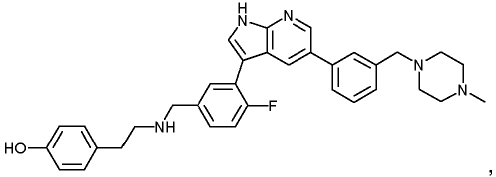 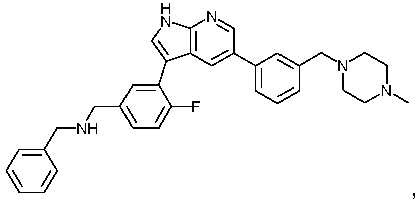 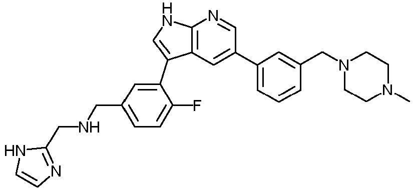 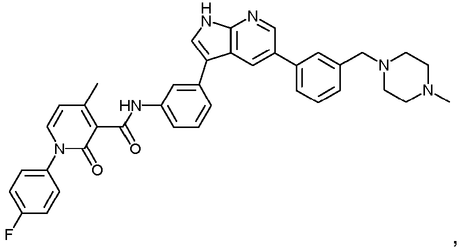 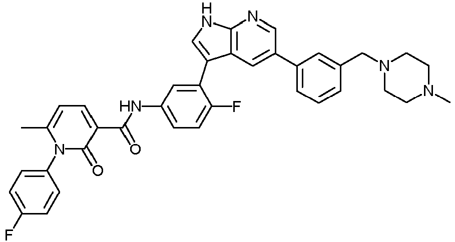 et

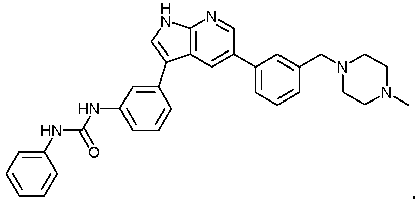

10. Composition pharmaceutique comprenant un composé selon l'une quelconque des revendications 1 à 9 ou un sel pharmaceutiquement acceptable de celui-ci en tant que principe actif, et un excipient pharmaceutiquement acceptable pour son utilisation dans la prévention et/ou le traitement des infections virales.

11. Composition pharmaceutique pour son utilisation selon la revendication 10, comprenant en outre un agent thérapeutique supplémentaire choisi parmi un agent antiviral, un agent anti-inflammatoire, un agent immunomodulateur ou immunosuppresseur, un agent pour le traitement de troubles d'immunodéficience et un agent pour le traitement de la douleur.

12. Composé pour son utilisation selon l'une quelconque des revendications 1 à 9 ou composition pour son utilisation selon la revendication 10 ou 11, dans lequel ou laquelle les infections virales sont dues aux Flavivirus, tels que les virus de la dengue, Zika, West Nile, Kunjin, aux Alphavirus, tels que les virus Chikungunya, Mayaro, Semliki Forest, Sindbis, le virus de l'encéphalite équine de l'Est, le virus de l'encéphalite equine Occidental, le virus de l'encéphalite équine du Venezuela, aux Filovirus tels que le virus Ebola, le virus de la fièvre de Marburg), aux arénavirus tels que le virus de la fièvre de Lassa, le virus Junin, le virus Amapari, aux picornavirus tels que le virus de la stomatite vésiculeuse, aux paramyxovirus, tels que le virus influenza) ou aux coronavirus tels que le SARS-COV2.

## Patentansprüche

1. Eine Verbindung der Formel (I): 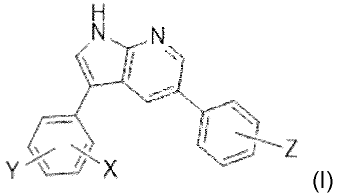 in dem
X stellt ein Wasserstoffatom oder ein Halogenatom dar,
Y ausgewählt ist aus einem Wasserstoffatom, einer -CN-, -CH₂OH-, - CH=NOH-, -CO₂H-Gruppe, einem gegebenenfalls einfach oder mehrfach substituierten Aryl, einem gegebenenfalls einfach oder mehrfach substituierten Heteroaryl und -L-(CH₂)p-W,
- L bedeutet -C(O)NH-, -CH₂NH-, -NHC(O)-, -CH₂N=CH-, -CH=N-, - NHC(O)NH- oder -CH₂NHC(O)-,
- p ist eine ganze Zahl von 0 bis 2,
- wenn L -CH₂NH-, -NHC(O)-, -CH₂N=CH-, -CH=N-, -NHC(O)NH- oder - CH₂NHC(O)- darstellt, ist W ausgewählt aus:
- ein Wasserstoffatom,
- ein C₁-C₆-Alkyl, vorzugsweise eine Methylgruppe,
- ein C₆-C₁₀-Aryl, gegebenenfalls ein- oder mehrfach substituiert, vorteilhafterweise durch 1 bis 3 Gruppen, unabhängig voneinander ausgewählt aus einem Halogenatom, einem Hydroxyl, einem C₁-C₆-Alkyl oder einem C₁-C₆-Alkoxyl, und
- ein 5- bis 10-gliedriges Heteroaryl, das 1 bis 3 Heteroatome enthält, die unabhängig voneinander aus N, O und S ausgewählt sind, wobei das Heteroaryl gegebenenfalls ein- oder mehrfach substituiert ist, vorteilhafterweise ist es gegebenenfalls mit 1 bis 4 Gruppen substituiert, die unabhängig voneinander ausgewählt sind aus
∘ ein Halogenatom,
∘ ein Hydroxyl,
∘ eine Oxo-Gruppe,
∘ ein C₁-C₆-Alkoxyl,
∘ ein C₁-C₆-Alkyl, in dem 1 oder mehrere Wasserstoffatome gegebenenfalls durch ein Fluoratom ersetzt sind, und
∘ ein C₆-C₁₀-Aryl, gegebenenfalls substituiert mit einem Halogenatom und/oder einem C₁-C₆-Alkyl;
- wenn L -C(O)NH- darstellt, ist W ausgewählt aus:
- ein gegebenenfalls ein- oder mehrfach substituiertes Phenyl, das vorteilhafterweise gegebenenfalls mit einem Halogenatom, einer Hydroxylgruppe, einer C₁-C₆-Alkyl- oder einer C₁-C₆-Alkoxylgruppe substituiert ist, und
- ein 5- bis 10-gliedriges Heteroaryl mit 1 bis 2 Heteroatomen, die unabhängig voneinander aus N, O und S ausgewählt sind, wobei das Heteroaryl gegebenenfalls ein- oder mehrfach substituiert ist, vorteilhafterweise ist es gegebenenfalls mit 1 bis 4 Substituenten substituiert, die unabhängig voneinander ausgewählt sind aus
∘ ein Halogenatom,
∘ ein Hydroxyl,
∘ eine Oxo-Gruppe,
∘ eine C₁-C₆-Alkoxylgruppe,
∘ ein C₁-C₆-Alkyl, in dem 1 oder mehrere Wasserstoffatome gegebenenfalls durch ein Fluoratom ersetzt sind, und
∘ ein C₆-C₁₀-Aryl, gegebenenfalls substituiert mit einem Halogenatom und/oder einem C₁-C₆-Alkyl;
- Z steht für -CH₂Q oder -O(CH₂)ₘT,
- Q ist -OH, -SO₂R₁, -NR₂R₃ oder -R₄
- R₁ stellt ein C₁-C₆-Alkyl dar,
- R₂ und R₃ stehen jeweils unabhängig voneinander für ein C₁-C₆-Alkyl, und
- R₄ ein 5-7-gliedriges Heterocycloalkyl darstellt, das 1 bis 2 Heteroatome umfasst, die unabhängig voneinander aus N, O und S ausgewählt sind, wobei das Heterocycloalkyl gegebenenfalls mit 1 bis 3 Substituenten substituiert ist, die unabhängig voneinander aus einem C₁-C₆-Alkyl, einem C₁-C₆-C(O)-Alkyl und einem C₁-C₆-C(O)O-Alkyl ausgewählt sind,
- m ist gleich 1 oder 2,
- T ist -O(CH₂)ₙCH₃ oder -R₅
- n ist 0 oder 1
- R₅ ein 5-7-gliedriges Heterocycloalkyl ist, das 1 bis 2 Heteroatome umfasst, die unabhängig voneinander aus N, O und S ausgewählt sind, wobei das Heterocycloalkyl gegebenenfalls mit 1 bis 3 Substituenten substituiert ist, die unabhängig voneinander aus einem C₁-C₆-Alkyl, einem C₁-C₆-C(O)-Alkyl und einem C₁-C₆-C(O)O-Alkyl ausgewählt sind;
oder ein pharmazeutisch annehmbares Salz davon oder ein Gemisch davon, zur Verwendung bei der Vorbeugung und/oder Behandlung von Virusinfektionen.

2. Die Verbindung zur Verwendung nach Anspruch 1, mit der Formel (Id): 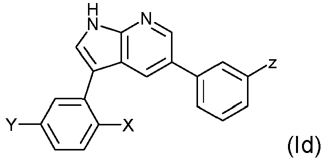 worin X, Y und Z wie in Anspruch 1 definiert sind.

3. Verbindung für ihre Verwendung nach Anspruch 1 oder 2, **dadurch gekennzeichnet, dass** Z -CH₂R₄ darstellt, wobei R₄ eine Piperazin-Gruppe darstellt, die gegebenenfalls mit einem C₁-C₆-Alkyl, insbesondere einer Methylgruppe, substituiert ist.

4. Verbindung zur Verwendung nach einem der Ansprüche 1 bis 3, **dadurch gekennzeichnet, dass** X ein Halogen, insbesondere Fluor, darstellt.

5. Verbindung zur Verwendung nach einem der Ansprüche 1 bis 4, **dadurch gekennzeichnet, dass** Y ein Aryl, ein Heteroaryl oder L-(CH₂)ₚ-W darstellt, wobei L -CH₂NH oder -NHC(O)NH- darstellt, p eine ganze Zahl von 0 bis 2 ist und W ausgewählt ist aus:
- eine Phenylgruppe, die gegebenenfalls mit einer Hydroxylgruppe oder einer C₁-C₆-Alkylgruppe substituiert ist, und
- ein 5- bis 10-gliedriges Heteroaryl mit 1 bis 2 Heteroatomen, die unabhängig voneinander aus N, O oder S ausgewählt sind.

6. Verbindung für ihre Verwendung nach Anspruch 5, **dadurch gekennzeichnet, dass** Y für L-(CH₂)ₚ-W steht, wobei L für -CH₂NH oder - NHC(O)NH- steht, p eine ganze Zahl von 0 bis 2 ist und W ausgewählt ist aus:
- eine gegebenenfalls mit einer Hydroxylgruppe substituierte Phenylgruppe und
- ein Imidazol.

7. Verbindung zur Verwendung nach einem der Ansprüche 1 bis 4, **dadurch gekennzeichnet, dass** Y -NHC(O)-W darstellt, wobei W eine 5- bis 10-gliedrige Heteroarylgruppe darstellt, die 1 bis 2 Heteroatome umfasst, die unabhängig voneinander aus N, O oder S ausgewählt sind, wobei das Heteroaryl gegebenenfalls mit 1 bis 4 Substituenten substituiert ist, die unabhängig voneinander ausgewählt sind aus:
- eine Oxo-Gruppe,
- eine C₁-C₆-Alkylgruppe, in der 1 oder mehrere Wasserstoffatome gegebenenfalls durch ein Fluoratom ersetzt sind, und
- eine C₆-C₁₀-Arylgruppe, gegebenenfalls substituiert mit einem Halogenatom und/oder einer C₁-C₆-Alkylgruppe.

8. Verbindung für ihre Verwendung nach Anspruch 7, **dadurch gekennzeichnet, dass** W 2(1H)-Pyridinon ist, das gegebenenfalls mit 1 bis 4 Substituenten substituiert ist, die unabhängig voneinander ausgewählt sind aus:
- ein Methyl, und
- ein gegebenenfalls mit einem Fluoratom substituiertes Phenyl.

9. Verbindung zur Verwendung nach Anspruch 1, ausgewählt aus: 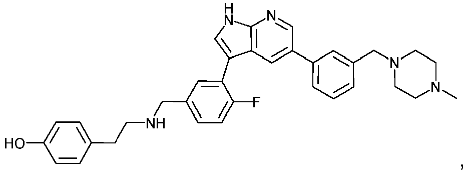 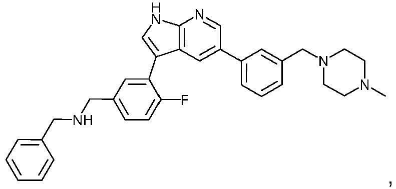 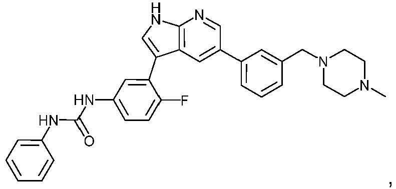 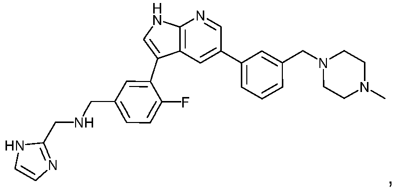 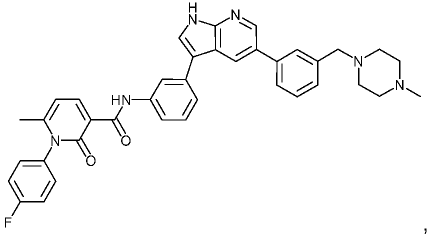 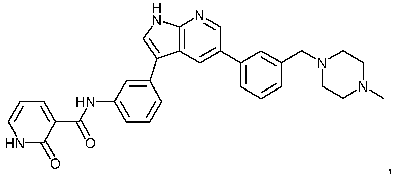 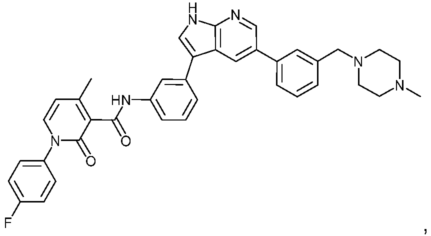 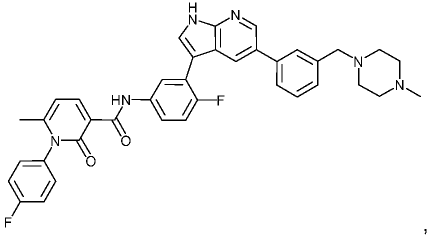 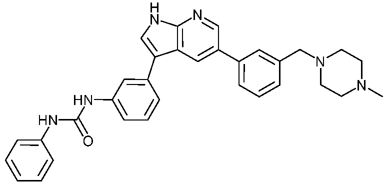 und 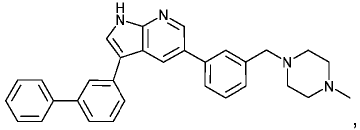 vorzugsweise von 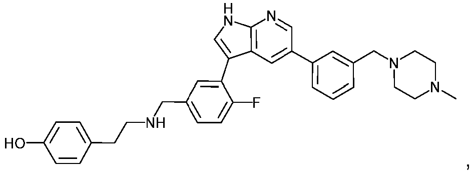 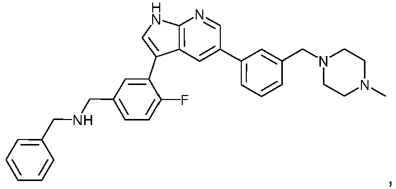 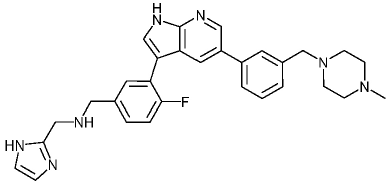 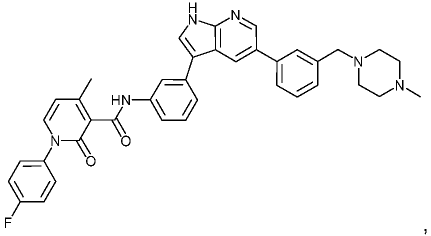 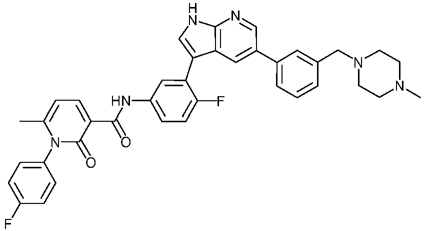 und

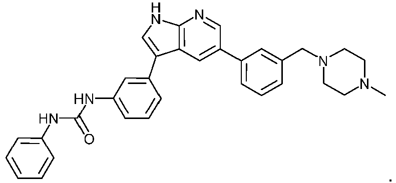

10. Pharmazeutische Zusammensetzung, umfassend eine Verbindung nach einem der Ansprüche 1 bis 9 oder ein pharmazeutisch annehmbares Salz davon als Wirkstoff und einen pharmazeutisch annehmbaren Hilfsstoff zur Verwendung bei der Vorbeugung und/oder Behandlung von Virusinfektionen.

11. Die pharmazeutische Zusammensetzung für die Verwendung nach Anspruch 10, die ferner ein zusätzliches therapeutisches Mittel enthält, das aus einem antiviralen Mittel, einem entzündungshemmenden Mittel, einem immunmodulatorischen oder immunsuppressiven Mittel, einem Mittel zur Behandlung von Immunschwächekrankheiten und einem Mittel zur Schmerzbehandlung ausgewählt ist.

12. Verbindung zur Verwendung nach einem der Ansprüche 1 bis 9 oder Zusammensetzung zur Verwendung nach Anspruch 10 oder 11, wobei die Virusinfektionen durch Flaviviren, wie Dengue-Virus, Zika-Virus, West-Nil-Virus, Kunjin-Virus, Alphaviren, wie Chikungunya-Virus, Mayaro-Virus, Semliki Forest-Virus, Sindbis-Virus, Östliches Pferdeenzephalitis-Virus, Westliches Pferdeenzephalitis-Virus, Venezolanisches Pferdeenzephalitis-Virus, Filoviren wie das Ebola-Virus, das Marburg-Fieber-Virus, Arenaviren wie das Lassa-Fieber-Virus, das Junin-Virus, das Amapari-Virus, Pikornaviren wie das Vesikuläre Stomatitis-Virus, Paramyxoviren wie das Influenza-Virus oder Coronaviren wie SARS-COV2.

## Claims

1. A compound of formula (I): 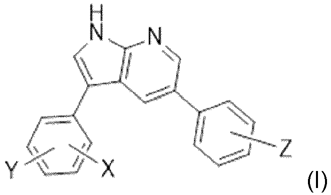 in which
X represents a hydrogen atom or a halogen atom,
Y is selected from a hydrogen atom, a -CN, -CH₂OH, -CH=NOH, -CO₂H group, an aryl optionally mono- or polysubstituted, a heteroaryl optionally mono- or polysubstituted, and -L-(CH₂)p-W,
- L represents -C(O)NH-, -CH₂NH-, -NHC(O)-, -CH₂N=CH-, -CH=N-, - NHC(O)NH- or -CH₂NHC(O)-,
- p is an integer from 0 to 2,
- when L represents -CH₂NH-, -NHC(O)-, -CH₂N=CH-, -CH=N-, -NHC(O)NH- or -CH₂NHC(O)-, W is selected from:
• a hydrogen atom,
• a C₁-C₆ alkyl, preferably a methyl,
• a C₆-C₁₀ aryl, optionally mono- or polysubstituted, advantageously by 1 to 3 groups independently selected from a halogen atom, a hydroxyl, a C₁-C₆ alkyl, or a C₁-C₆ alkoxyl, and
• a 5- to 10-membered heteroaryl containing from 1 to 3 heteroatoms independently selected from N, O and S, said heteroaryl optionally being mono- or polysubstituted, advantageously it is optionally substituted with 1 to 4 groups independently selected from
∘ a halogen atom,
∘ a hydroxyl,
∘ an oxo group,
∘ a C₁-C₆ alkoxyl,
∘ a C₁-C₆ alkyl, wherein 1 or several hydrogen atoms is optionally replaced with a fluorine atom, and
o a C₆-C₁₀ aryl, optionally substituted with a halogen atom and/or a C₁-C₆ alkyl;
- when L represents -C(O)NH-, W is selected from:
• an optionally mono- or polysubstituted phenyl, advantageously it is optionally substituted with a halogen atom, a hydroxyl group, a C₁-C₆ alkyl, or a C₁-C₆ alkoxyl group, and
• a 5- to 10-membered heteroaryl comprising from 1 to 2 heteroatoms independently selected from N, O and S, said heteroaryl optionally being mono- or polysubstituted, advantageously it is optionally substituted with 1 to 4 substituents independently selected from
∘ a halogen atom,
∘ a hydroxyl,
∘ an oxo group,
∘ a C₁-C₆ alkoxyl group,
∘ a C₁-C₆ alkyl, wherein 1 or several hydrogen atoms is optionally replaced with a fluorine atom, and
∘ a C₆-C₁₀ aryl, optionally substituted with a halogen atom and/or a C₁-C₆ alkyl;
- Z represents -CH₂Q or -O(CH₂)ₘT,
- Q represents -OH, -SO₂R₁, -NR₂R₃, or -R₄
- R₁ represents a C₁-C₆ alkyl,
- R₂ and R₃ each independently represent a C₁-C₆ alkyl, and
- R₄ represents a 5-7 membered heterocycloalkyl, comprising 1 to 2 heteroatoms independently selected from N, O and S, said heterocycloalkyl optionally substituted with 1 to 3 substituents independently selected from a C₁-C₆ alkyl, a C₁-C₆ C(O)-alkyl and a C₁-C₆ C(O)O-alkyl,
- m is equal to 1 or 2,
- T is -O(CH₂)ₙCH₃ or -R₅
• n is 0 or 1
• R₅ is a 5-7 membered heterocycloalkyl, comprising 1 to 2 heteroatoms independently selected from N, O and S, said heterocycloalkyl optionally substituted with 1 to 3 substituents independently selected from a C₁-C₆ alkyl, a C₁-C₆ C(O)-alkyl and a C₁-C₆ C(O)O-alkyl;
or a pharmaceutically acceptable salt thereof or a mixture thereof, for use in the prevention and/or treatment of viral infections.

2. The compound for its use according to claim 1, of formula (Id): 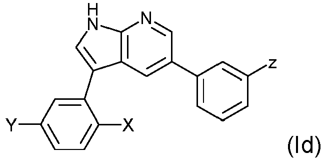 wherein X, Y and Z are as defined in claim 1.

3. The compound for its use according to claim 1 or 2, **characterized in that** Z represents -CH₂R₄ wherein R₄ represents a piperazine group, optionally substituted with a C₁-C₆, alkyl, in particular a methyl.

4. The compound for its use according to any one of claims 1 to 3, **characterized in that** X represents a halogen, in particular fluorine.

5. The compound for its use according to any one of claims 1 to 4, **characterized in that** Y represents an aryl, a heteroaryl or L-(CH₂)ₚ-W, L representing -CH₂NH or -NHC(O)NH-, p being an integer from 0 to 2, and W being selected from:
- a phenyl optionally substituted with a hydroxyl group, or a C₁-C₆ alkyl group, and
- a 5- to 10-membered heteroaryl comprising from 1 to 2 heteroatoms independently selected from N, O or S.

6. The compound for its use according to claim 5, **characterized in that** Y represents L-(CH₂)ₚ-W, L representing -CH₂NH or -NHC(O)NH-, p being an integer from 0 to 2, and W being selected from:
- a phenyl optionally substituted with a hydroxyl group, and
- an imidazole.

7. The compound for its use according to any one of claims 1 to 4, **characterized in that** Y represents -NHC(O)-W, with W representing a 5- to 10-membered heteroaryl group, comprising from 1 to 2 heteroatoms independently selected from N, O or S, said heteroaryl being optionally substituted with 1 to 4 substituents independently selected from:
- an oxo group,
- a C₁-C₆ alkyl group, wherein 1 or several hydrogen atoms is optionally replaced with a fluorine atom, and
- a C₆-C₁₀ aryl group, optionally substituted with a halogen atom and/or a C₁-C₆ alkyl group.

8. The compound for its use according to claim 7, **characterized in that** W is 2(1H)- pyridinone, optionally substituted with 1 to 4 substituents independently selected from:
- a methyl, and
- a phenyl optionally substituted with a fluorine atom.

9. A compound for its use according to claim 1, selected from: 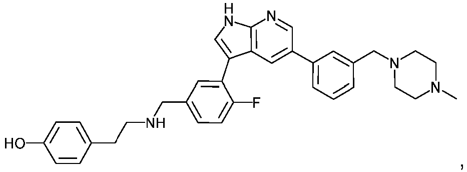 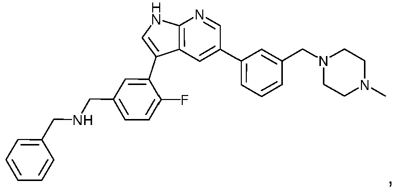 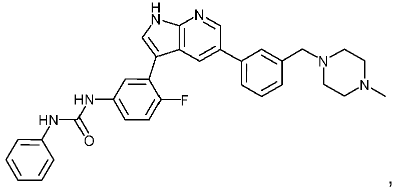 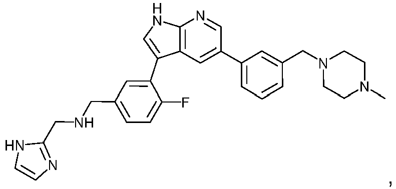 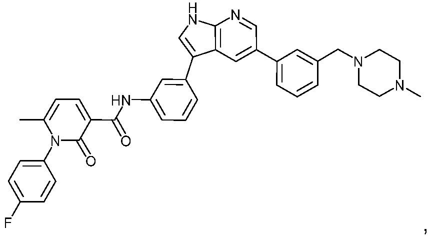 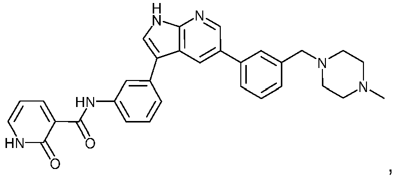 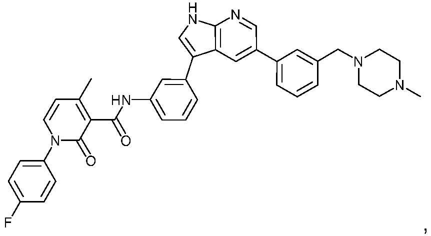 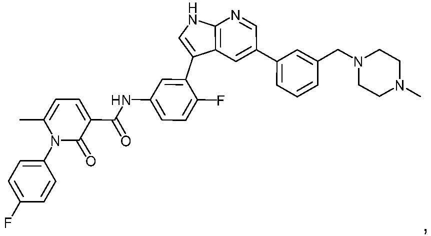 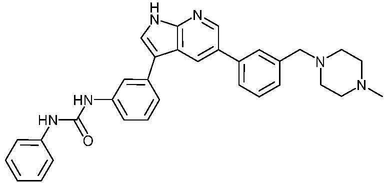 and 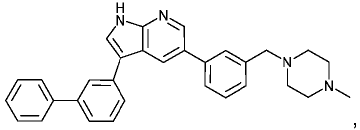 preferably from 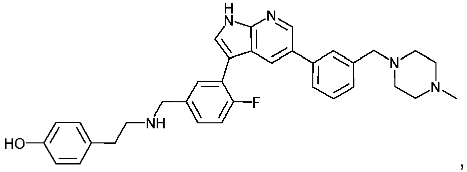 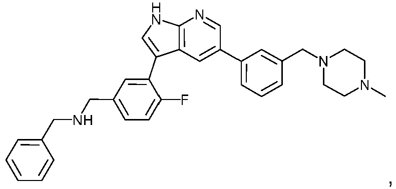 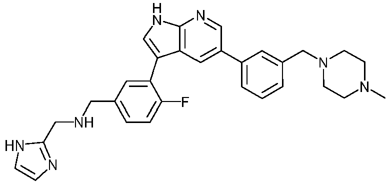 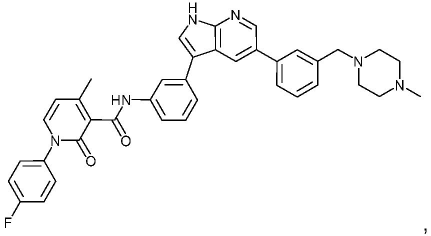 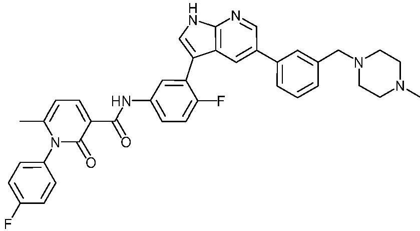 and

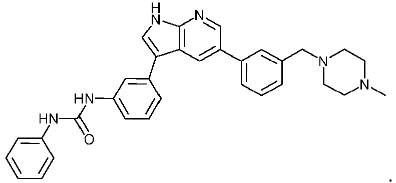

10. A pharmaceutical composition comprising a compound according to any one of claims 1 to 9 or a pharmaceutically acceptable salt thereof as an active ingredient, and a pharmaceutically acceptable excipient for use in the prevention and/or treatment of viral infections.

11. The pharmaceutical composition for its use according to claim 10, further comprising an additional therapeutic agent, chosen from an antiviral agent, an antiinflammatory agent, an immunomodulatory or immunosuppressive agent, an agent for the treatment of immunodeficiency disorders and an agent for pain treatment.

12. A compound for its use according to any one of the claims 1 to 9 or composition for its use according to claim 10 or 11, wherein the viral infections are due to Flaviviruses, such as dengue virus, Zika virus, West Nile virus, Kunjin virus, Alphaviruses, such as Chikungunya virus, Mayaro virus, Semliki Forest virus, Sindbis virus, Eastern equine encephalitis virus, Western equine encephalitis virus, Venezuelan equine encephalitis virus, Filoviruses such as Ebola virus, Marburg fever virus, arenaviruses such as Lassa fever virus, Junin virus, Amapari virus, picornaviruses such as vesicular stomatitis virus, paramyxoviruses such as influenza virus or coronaviruses such as SARS-COV2.
